(19) **Europäisches Patentamt European Patent Office Office européen des brevets**

(11) **EP 1 625 398 B1**

(12) **FASCICULE DE BREVET EUROPEEN**

(45) Date de publication et mention
de la délivrance du brevet:
**22.10.2008 Bulletin 2008/43**

(51) Int Cl.:
*G01N 33/53* (2006.01)   *G01N 33/543* (2006.01)
*B01J 19/00* (2006.01)

(21) Numéro de dépôt: **04742868.5**

(86) Numéro de dépôt international:
**PCT/FR2004/050195**

(22) Date de dépôt: **17.05.2004**

(87) Numéro de publication internationale:
**WO 2004/104580 (02.12.2004 Gazette 2004/49)**

(54) **DISPOSITIF ET PROCEDES D'ACCROCHAGE/DECROCHAGE D'UNE CIBLE OU D'UN OBJET PRESENT DANS UN ECHANTILLON**

VORRICHTUNG UND VERFAHREN ZUR BINDUNG UND/ODER AUFTRENNUNG EINER ZIELMOLEKÜL ODER EINES OBJEKTES AUS EINER PROBE

DEVICE AND METHODS FOR COUPLING/ UNCOUPLING A TARGET OR AN OBJECT PRESENT IN A SAMPLE

(84) Etats contractants désignés:
**AT BE BG CH CY CZ DE DK EE ES FI FR GB GR HU IE IT LI LU MC NL PL PT RO SE SI SK TR**

(30) Priorité: **21.05.2003 FR 0350166**

(43) Date de publication de la demande:
**15.02.2006 Bulletin 2006/07**

(73) Titulaire: **COMMISSARIAT A L'ENERGIE ATOMIQUE**
**75015 Paris (FR)**

(72) Inventeurs:
• **ESCOFFIER, Céline**
**F-21000 Dijon (FR)**
• **MARCHAND, Gilles**
**F-38350 LA MURE D'ISERE (FR)**
• **REVOL-CAVALIER, Frédéric**
**F-38180 SEYSSINS (FR)**
• **VINET, Françoise**
**F-38000 GRENOBLE (FR)**

(74) Mandataire: **Poulin, Gérard et al**
**Brevalex**
**3, rue du Docteur Lancereaux**
**75008 Paris (FR)**

(56) Documents cités:
**WO-A-99/29711**   **US-A- 6 113 768**
**US-B1- 6 280 595**   **US-B1- 6 514 762**

**EP 1 625 398 B1**

## Description

## DOMAINE TECHNIQUE

La présente invention se rapporte à un dispositif et à des procédés d'accrochage ou de décrochage d'une cible ou d'un objet présent dans un échantillon sur une sonde fixée sur un support.

La cible peut par exemple être choisie dans le groupe constitué d'une molécule chimique ou biologique, une cellule, d'une bactérie, d'une particule fonctionnalisée, telle qu'une bille de latex ou une bille de verre, d'une protéine, d'un acide désoxyribonucléotidique (ADN ou ADNc), d'un oligonucléotide, d'un acide ribonucléotidique, d'un acide nucléique de peptide (« PNA » pour « peptide nucleic acid » en anglais), d'une enzyme, d'une molécule à transfecter, d'un principe actif par exemple d'intérêt pharmacologique, etc. La sonde peut être par exemple une molécule chimique ou biologique ou un objet biologique capable de se lier à la fois au support et à la cible. L'objet peut être un des éléments précités, il est « porté » par la cible.

L'invention trouve des applications dans de très nombreux domaines par exemple dans des procédés de séparation ou purification de molécules ou d'objets biologiques, dans des procédés de concentration de molécules ou d'objets biologiques, dans des procédés de détection, etc.

L'invention s'applique à l'ensemble des microsystèmes utilisant l'accrochage et le décrochage de cibles ou d'objets biologiques et/ou chimiques. Elle peut s'appliquer notamment aux microsystèmes pour la biologie, par exemple aux puces à ADN, aux puces de tri cellulaire ; aux microsystèmes chimiques, par exemple activation de fonctionnalisation ou puces de détection ; ou de chimie combinatoire, par exemple ciblage de molécules actives.

## ETAT DE LA TECHNIQUE ANTERIEURE

Dans le texte ci-dessous, les références entre crochets [ ] renvoient à la liste de références annexée.

La plupart des systèmes et techniques actuels utilisent des processus autres que l'électrochimie. Typiquement, ces techniques exploitent soit des interactions physiques telles que des interactions électrostatiques, hydrophile/hydrophobe, stériques, topographiques, physisorption, etc., soit dépendent d'un processus chimique. Quelques systèmes font appel à des procédés que l'on peut qualifier « d'actifs » car une commande électrique, électrochimique ou photochimique, intervient dans la modulation des propriétés de surface. De manière générale, la combinaison accrochage/décrochage des molécules par ces systèmes n'est pas réversible ni contrôlable car ceux-ci sont souvent passifs et/ou non spécifiques.

Parmi les systèmes d'accrochage et de décrochage de molécules sur une surface, adaptés à des applications biologiques et/ou chimiques, certain utilisent des modifications des propriétés de surface.

On peut citer par exemple l'altération électrochimique d'un groupement actif présent sur une surface suivie d'une réaction chimique : protonation/déprotonation de groupes acides et/ou bases en terminaison de couches auto assemblées de thiols, couples redox de type $X^{(-)}/XH$, en surface ou en solution, pour provoquer une déprotection. De tels systèmes sont décrits par exemples dans les références [1] et [2] citées en annexe. Dans ces systèmes, les couples redox utilisés sont relativement complexes et les solvants employés pour la synthèse chimique sont le plus souvent non aqueux ce qui conduit inévitablement à une limitation du spectre d'utilisation. Il s'agit par exemple d'une réduction ou oxydation électrochimique conduisant à une réaction chimique, par exemple, réduction d'une quinone en hydroquinone suivie d'une lactonisation (décrochage de ligands ou de cellules) ou encore oxydation d'une hydroquinone en quinone suivie d'une réaction de Diels-Alder (immobilisation de ligands ou de cellules) comme cela est décrit dans la référence [2].

Ces techniques, en partie électrochimiques, ne permettent malheureusement pas de disposer d'un accrochage réversible puisqu'elles sont couplées à une réaction chimique irréversible : chimie de synthèse comme le montre le document de référence [3].

On peut citer également les systèmes utilisant l'activation photochimique d'un groupement présent sur une surface tels que ceux décrits dans les références [2] et [4]. Ces systèmes utilisent un changement de la conformation du groupement par isomérisation induit par voie photonique et une réaction avec, ou une reconnaissance par, le produit photo activé tel qu'une enzyme, un anticorps, un ligand ou des groupements chimiques [5].

L'accrochage des objets, dans ce cas, peut malheureusement conduire à un accrochage non spécifique [2] et nécessite un banc optique et un système d'activation complexe et délicat à mettre en oeuvre.

On peut citer aussi les techniques utilisant un changement de comportement hydrophile / hydrophobe d'une surface recouverte de polymères particuliers (« LCST » : « Lower Critical Solution Temperature ») comme cela est décrit dans le document [6]. Ces polymères passent d'un état à l'autre si leur température est inférieure ou supérieure à une température critique (vers 37°C). Ceci permet, par attraction/répulsion hydrophile/ hydrophobe d'accrocher ou de décrocher des objets (ayant des propriétés hydrophiles ou hydrophobes) par refroidissement (ou réchauffement).

Cependant, pour des objets de grande taille à l'échelle des microsystèmes, par exemple des cellules, le détachement s'accompagne d'une importante inertie de réaction. En outre, l'intégration de ces polymères à un microsystème nécessite encore de nombreux efforts de recherche et d'adaptation aux procédés de micro technologies [7].

On peut aussi citer les systèmes utilisant la fonctionnalisation d'une surface avec des groupements pouvant effectuer une reconnaissance chimique et/ou stérique avec un autre groupement présent sur l'objet à ancrer [8], [9] et [10] . Ces systèmes immobilisent des fonction chimiques ou biologiques telles que des groupements acide carboxyle, amine, hydroxyle, etc. ou oligonucléotides, par couches auto assemblées de thiols (« SAM ») fonctionnalisés, par silanisation ou par polymères conducteurs fonctionnalisés, par greffage de complexes métallo-organiques, ou encore de molécules cages [11].

Malheureusement, dans ces systèmes, l'accrochage et le décrochage ne peuvent être ni commandés ni effectués localement. Ces systèmes manquent donc de précision.

On peut citer également les systèmes utilisant une action électrique, électrostatique comme cela est décrit dans la référence [12]. Ces systèmes utilisent par exemple un champ électrique pour séparer deux parties d'un assemblage moléculaire porteur de charges [13], une désorption cathodique (répulsion électrostatique) [14] ou un accrochage ou décrochage d'objets chargés pour maintenir l'électroneutralité de la zone d'accrochage ou de décrochage ou à l'intérieur d'une membrane (volume).

Ces techniques n'offrent cependant pas de réelle spécificité et tout objet porteur de charge sera simultanément attiré/repoussé. La réversibilité de cette approche vis-à-vis d'objets complexes et de volume important à l'échelle des microsystèmes, par exemple, une cellule, une bactérie, n'est pas acquise. Dans le cas du maintien de l'électroneutralité d'une membrane, la taille des objets pouvant être immobilisés est un facteur limitant.

Il existe donc un réel besoin d'un système qui ne présente pas les nombreux problèmes des systèmes précités de l'art antérieur.

**Exposé de l'invention**

Le système de la présente invention, sous la forme d'un dispositif et de procédés utilisant ce dispositif, permet précisément d'apporter une solution à ces nombreux problèmes de l'art antérieur. En particulier, il utilise des solutions aqueuses, il peut être réversible, spécifique, précis, reproductible, simple à mettre en oeuvre, il permet de s'adapter aussi bien à l'accrochage de cibles ou d'objets de grande taille tels que des cellules qu' à des cibles ou objets de petite taille tels que des molécules chimiques, il s'adapte aisément aux procédés de microtechnologies, et il permet une commande et une mise en oeuvre locale. En outre, l'activation du dispositif pour l'accrochage/décrochage possède une faible inertie de réaction.

Dans la suite de la description, le terme « cible » sera réservé aux molécules ou objets qui se lient directement à la sonde pour former une liaison sonde-cible. Le terme « objet » sera réservé aux molécules ou objets qui sont accrochés sur la sonde par l'intermédiaire de la « cible » en formant avec ladite cible une liaison objet-cible. Autrement dit l'objet ne se lie pas directement à la sonde, mais par l'intermédiaire de la cible qui est reconnue par la sonde.

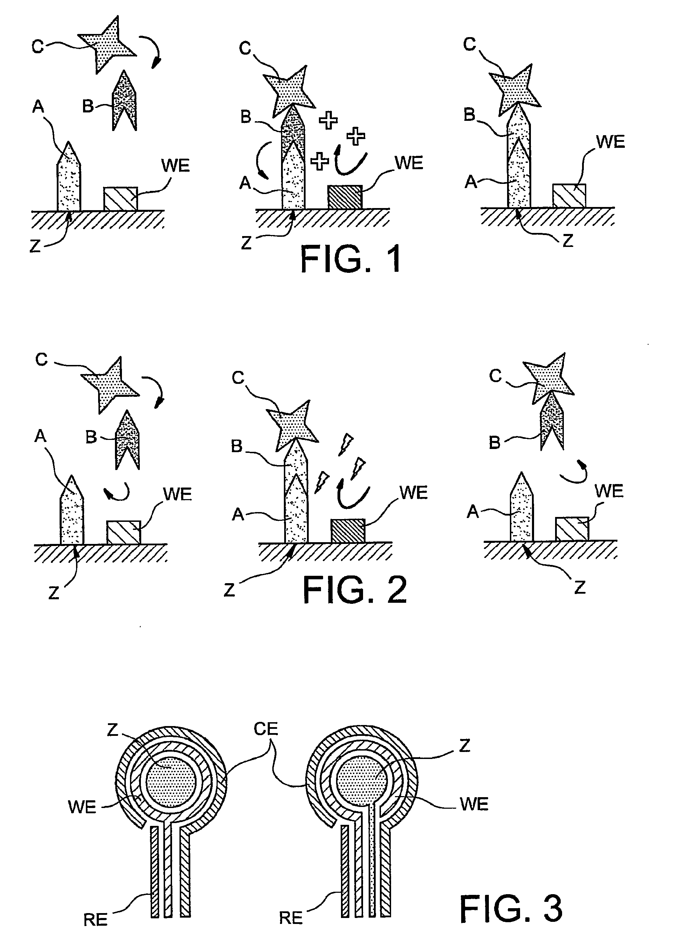

Les figures 1 et 2 annexées représentent schématiquement un dispositif selon l'invention.

Le dispositif de la présente invention est un dispositif permettant l'accrochage ou le décrochage localisé d'une cible (B) sur une sonde (A) fixée sur un support.

Il comprend :

- un support ayant une surface comportant une zone d'accrochage (Z) susceptible d'être fonctionnalisée par une sonde (A) capable de se lier à une cible (B) pour l'accrocher ;
- une électrode de travail (WE) et une contre électrode (CE) de cette électrode de travail disposées sur le support au voisinage de la zone d'accrochage dans lequel l'électrode de travail borde ou entoure la zone d'accrochage ;
- des moyens permettant d'appliquer un courant électrique ou un potentiel déterminé à ladite électrode de travail de manière à provoquer, lorsque ladite zone d'accrochage et lesdites électrodes sont immergées dans une solution aqueuse, une variation locale de pH au niveau de ladite zone d'accrochage; ladite zone d'accrochage (Z) et ladite électrode de travail (WE) étant séparées l'une de l'autre par un espace vide.

Le procédé de l'invention est, suivant un premier mode de réalisation, un procédé d'accrochage d'une cible (B) présente dans un échantillon aqueux à une sonde (A), ledit procédé comprenant les étapes suivantes :

a) mise en contact de l'échantillon aqueux avec la zone d'accrochage d'un dispositif selon l'invention, ladite zone d'accrochage étant fonctionnalisée par la sonde (A) capable de se lier, en fonction du pH, à la cible (B) pour l'accrocher ;

b) application d'un courant électrique ou d'un potentiel à l'électrode de travail dudit dispositif de façon à modifier localement, au niveau de ladite zone d'accrochage, le pH de l'échantillon aqueux pour que la sonde reconnaisse et se lie spécifiquement à la cible pour l'accrocher.

Le procédé de l'invention est, suivant un deuxième mode de réalisation, un procédé d'accrochage et de décrochage d'une cible (B) présente dans un échantillon aqueux sur une sonde (A), ledit procédé comprenant les étapes suivantes :

a') mise en contact de l'échantillon aqueux comprenant la cible (B) avec la zone d'accrochage d'un dispositif selon la présente invention, ladite zone d'accrochage étant fonctionnalisée par la sonde (A), pour que la cible (B) s'accroche à ladite sonde ;

b') application d'un courant électrique ou d'un potentiel à l'électrode de travail dudit dispositif de façon à modifier localement, au niveau de ladite zone d'accrochage, le pH de la solution de travail pour que la cible (B) se décroche de la sonde (A).

L'échantillon aqueux est une solution aqueuse comprenant la cible à accrocher sur la sonde. Il provient par exemple d'un simple mélange de la cible à une solution aqueuse, ou d'un prélèvement effectué sur un animal, une plante, dans un milieu de culture, dans un réacteur de culture biologique (culture cellulaire, levures, champignons, algues, enzymes, etc.), dans un réacteur chimique, d'un gaz (par exemple l'air ambiant) ou à partir d'un effluent industriel liquide ou gazeux. Si ce prélèvement ne permet pas de mettre en oeuvre le procédé de l'invention, par exemple du fait de sa nature (gaz, solide), de sa concentration ou des éléments qu'il contient (résidus solides, déchets, suspension, molécules interférentes, etc.) le procédé de l'invention comprend en outre une étape préalable de mise en solution aqueuse du prélèvement par les techniques connues de l'homme du métier. L'essentiel est que l'échantillon auquel est appliqué le procédé de l'invention soit aqueux.

En effet, dans la présente invention, l'eau présente dans l'échantillon peut subir soit une oxydation soit une réduction suivant les conditions électrochimiques (courant ou potentiel) appliquées à l'électrode de travail. Les couples mis en jeu, $O_2/H_2O$ et $H_2O/H_2$, ont des potentiels respectifs déterminés par $E_1 = 1, 23 - 0,06pH$, et $E_2 = - 0,06pH$. Le pH initial de la solution de travail, ainsi que l'immunité cinétique des réactions, dépendant de l'électrolyte et des électrodes utilisées, détermine le potentiel de travail permettant d'obtenir la variation de pH désirée. La zone d'immunité cinétique est la zone de potentiel supérieure au potentiel théorique (donné par les équations ci-dessus) dans laquelle aucune réaction ne se produit, contrairement à ce que prédit la théorie. Il est donc nécessaire d'appliquer une surtension pour vaincre l'immunité cinétique. Par exemple, sur électrode de platine, la surtension cathodique de - 0,2 V et la surtension anodique de 0,6 V.

Dans la présente invention, le processus d'accrochage implique la formation d'un pont chimique entre la sonde et sa cible : soit par une variation locale du pH au moyen du dispositif de l'invention, le milieu au voisinage de la zone d'accrochage étant rendu localement acide ou basique par activation électrochimique suivant le premier mode de réalisation de l'invention (« accrochage actif »); soit spontanément, par exemple du fait du pH de la solution aqueuse de travail, du fait d'une affinité chimique ou biologique entre la sonde et la cible, du fait d'une reconnaissance stérique, etc., lors de la mise en contact sonde-cible suivant le deuxième mode de réalisation de l'invention (« accrochage passif »). La formation de ce pont chimique s'effectue entre deux groupements, dont l'un appartient à la sonde immobilisée à la surface de la zone d'accrochage, et l'autre à la cible.

Ainsi, suivant le choix du mode de réalisation de l'invention, lorsque la cible est présente dans l'échantillon, elle est accrochée par la sonde sur la zone d'accrochage soit grâce à la variation locale du pH induite au niveau de ladite zone d'accrochage par la microcellule électrochimique, soit spontanément.

Dans le premier mode de réalisation de l'invention, et lorsque la liaison sonde-cible est réversible, par exemple en fonction du pH, la cible peut être libérée de la sonde par exemple en appliquant une étape consistant à interrompre l'application du courant électrique ou du potentiel à l'électrode de travail ou à appliquer un courant électrique ou un potentiel différent à ladite électrode de travail de façon à modifier localement le pH de la solution de travail ou d'une solution de rinçage afin que la cible se décroche de la sonde. Dans ce cas, le procédé de l'invention comprend donc un accrochage et un décrochage « actif » de la cible. Par exemple, on peut appliquer à l'électrode de travail un courant ou un potentiel permettant de revenir à la valeur initiale de pH ou à une valeur permettant la libération de la cible.

Dans le premier mode de réalisation de l'invention, et lorsque la liaison sonde/cible est réversible, la cible peut aussi être libérée en utilisant une solution de rinçage qui rompt la liaison sonde-cible, par exemple du fait du pH ou de la nature chimique de cette solution de rinçage.

Une ou plusieurs étapes de rinçage de la zone d'accrochage, avant et/ou après l'étape b) peut (peuvent) également être réalisée(s), ceci par exemple à des fins de purification de la cible fixée sur la sonde. La solution de rinçage est alors bien entendu de préférence une solution qui préserve la liaison sonde-cible. Cette solution peut par exemple être identique à la solution aqueuse qui a permis la préparation de l'échantillon.

Selon une variante du deuxième mode de réalisation, l'accrochage de la cible par la sonde dans l'étape a') peut être réalisé par application d'un courant électrique ou d'un potentiel à l'électrode de travail dudit dispositif de façon à modifier localement, au niveau de ladite zone d'accrochage, le pH de la solution de travail pour que la cible (B) s'accroche à ladite sonde (A). Dans ce cas, l'accrochage et le décrochage de la cible sont dits « actifs ». Suivant cette variante, l'accrochage peut être réalisé suivant le premier mode de réalisation de l'invention, et le décrochage peut être réalisé suivant le deuxième mode de réalisation de l'invention.

Les documents précités de l'art antérieur montrent qu'une approche purement électrochimique conforme à la présente invention, c'est-à-dire sans couplage avec un autre type de réaction non réversible, n'a jamais été abordée dans les techniques de l'art antérieur dans le but de contrôler et de faire varier les propriétés d'accrochage et/ou de décrochage d'une cible sur une surface fonctionnalisée par une sonde, par exemple d'un microsystème.

Le dispositif peut avantageusement être sous la forme d'un microsystème comportant un ou plusieurs dispositif(s) selon l'invention. Chaque dispositif forme une véritable microcellule électrochimique comprenant au moins deux électrodes : une électrode de travail et une contre-électrode ; et éventuellement une électrode de référence (RE) ; ainsi qu'une zone d'accrochage et/ou de décrochage. Cette dernière peut elle aussi être une électrode, par exemple lorsque sa fonctionnalisation avec la sonde est réalisée de manière électrique ou électrochimique, par exemple par électrogreffage.

Selon l'invention, une microcellule électrochimique pour une zone d'accrochage et/ou de décrochage est préférable afin d'obtenir une bonne localisation de la variation de pH, et donc un accrochage bien localisé des cibles. Ainsi, le dispositif de l'invention permet de mettre en oeuvre un procédé d'accrochage et/ou de décrochage localisé dans lequel il est possible de choisir spécifiquement, précisément, et indépendamment les unes des autres, la (ou les) zone(s) où une cible est à accrocher ou à décrocher, parmi une matrice de plusieurs zones potentielles d'accrochage et/ou de décrochage réparties à la surface d'un microsystème et fonctionnalisées avec une ou plusieurs sondes identiques ou différentes.

Le support, sur lequel le dispositif de la présente invention peut être fabriqué, peut être un quelconque support permettant de mettre en oeuvre la présente invention. Il peut s'agir par exemple d'un support de biopuce tel que ceux classiquement utilisés, par exemple en silicium, en verre, en polymère, en métal, en plastique, etc. Des supports utilisables dans la présente invention sont décrits par exemple dans les documents référencés [15] et [16] dans la liste de référence.

La zone d'accrochage et/ou de décrochage peut être avantageusement constituée d'un matériau conducteur si une fonctionnalisation électrique ou électrochimique est nécessaire. Elle peut être constituée de tout autre matériau utilisable pour greffer la sonde si d'autres techniques de fonctionnalisation sont choisies, par exemple des techniques de fonctionnalisation chimiques. Il peut s'agir d'un matériau modifié chimiquement ou biologiquement pour que la sonde puisse être fixée dessus. Il peut s'agir de la surface même du support ou d'un revêtement, déposé sur ce support par les techniques habituelles de dépôt connues de l'homme du métier, permettant la fonctionnalisation par la sonde. Ce revêtement peut être par exemple du Si, du verre, du $SiO_2$ (permettant une silanisation), un polymère ou un copolymère conducteur approprié tels que ceux utilisés pour la fabrication de biopuces, en particulier pour la fixation de sondes moléculaires de biopuces, par exemple du polypyrrole, un métal, tel que Au, Ag, Pt, par exemple pour réaliser un électrogreffage, pour la formation de monocouches auto assemblées, etc. La zone d'accrochage peut être délimitée par exemple par la localisation des sondes qui la fonctionnalise, et par le voisinage des électrodes.

Le dispositif de la présente invention peut se présenter, par exemple à des fins de commercialisation, comme un substrat comportant une zone d'accrochage non fonctionnalisée et les électrodes. L'utilisateur de ce dispositif peut alors aisément, au moyen des techniques classiques de fonctionnalisation de surface de biopuces, fonctionnaliser cette zone avec une sonde qu'il a choisie en fonction de la cible qu'il désire accrocher pour obtenir un dispositif permettant de mettre en oeuvre un des procédés de l'invention.

Le dispositif de la présente invention peut aussi se présenter, à des fins de commercialisation, comme comportant déjà sa zone d'accrochage fonctionnalisée par une sonde (A) capable de se lier, en fonction du pH, à la cible (B) pour l'accrocher. Il permet alors de mettre en oeuvre un des procédés de l'invention immédiatement, sans fonctionnalisation préalable de la zone d'accrochage/décrochage, pour la cible correspondant à ladite sonde.

De manière générale, la fonctionnalisation de la zone d'accrochage par la sonde, qui consiste en une immobilisation de la sonde sur la zone d'accrochage, peut être réalisée au moyen des techniques habituelles de greffage chimiques ou électrochimiques (« électrogreffage »), par exemple telles que celles décrites dans les documents référencés [8] à [12] et [17] et [18] dans la liste de référence annexée.

Les figures 1 et 2 annexées représentent une zone d'accrochage (Z) fonctionnalisée par une sonde « A », par exemple biologique ou chimique.

La sonde est de manière générale choisie en fonction de la cible. La sonde « A » et la cible « B » (voir figures 1 et 2) sont composées d'un ou plusieurs éléments ayant de préférence des liaisons stables en pH et possédant des groupements chimiques pouvant interagir et s'accrocher en présence d'un pH acide ou basique lors de l'accrochage par voie électrochimique. Par exemple, la sonde « A » peut être porteur d'un (ou plusieurs) groupement (s) électrophile(s) pouvant réagir, en milieu acide ou basique, avec un (ou plusieurs) groupement(s) nucléophile(s) porté(s) par la cible « B » et vice versa. La liaison entre la sonde « A » et la cible « B » peut également consister en la formation d'un (de) pont(s) disulfure(s) par acidification du milieu suivant la réaction

$$\text{A-SH} + \text{HS-B} \ \mu \ \text{A-S-S-B}$$

Ainsi, selon l'invention, la sonde peut être choisie par exemple telle qu'elle est capable de se lier à la cible pour l'accrocher au moyen d'un groupement électrophile, par exemple choisi parmi des fonctions aldéhyde, halogénure, thiocyanate, isocyanate, ester activé, carbamate, époxyde, etc.

La sonde peut aussi être choisie telle qu'elle est capable de se lier à la cible pour l'accrocher au moyen d'un groupement nucléophile, par exemple choisi parmi les fonctions amine, alcoolate, phénol, phénate, oxyamine, hydrazine, etc. Selon l'invention, la sonde peut être choisie par exemple de manière à ce qu'elle puisse former, dans la solution de travail, avec la molécule cible pour l'accrocher, une liaison choisie parmi une liaison hydrogène, peptidique, amide, sulfonamide, ester d'acide carboxylique, ester d'acide sulfonique, silanoate substitué.

De manière générale, selon l'invention, la sonde peut être choisie parmi un acide désoxyribonucléotidique (ADN ou ADNc), un oligonucléotide, un acide ribonucléotidique, un acide nucléique de peptide (PNA), une protéine, un enzyme, un substrat d'enzyme, un récepteur hormonal, une hormone, un anticorps, un antigène, une cellule eucaryote ou procaryote ou des fragments de telles cellules, une algue, un champignon microscopique, etc. Le choix se fait en fonction de la cible. Par exemple, dans le groupe précité, la cible peut être un oligonucléotide complémentaire à l'oligonucléotide sonde, un substrat d'enzyme, un anticorps spécifique d'un antigène, etc.

La localisation de l'accrochage de la cible sur la sonde de la zone d'accrochage peut être déterminée par un accrochage spécifique lorsque l'assemblage sonde-cible possède une complémentarité unique. Il peut s'agir par exemple d'un accrochage sonde-cible qui exploite la complémentarité de séquence de deux brins oligonucléotidiques, par exemple d'ARN ou d'ADN, un des deux brins étant fixé sur le support pour former la zone d'accrochage fonctionnalisée, l'autre brin constituant la cible à accrocher. La liaison entre la sonde et la cible se fait alors sous la forme d'un appariement des bases complémentaires des deux brins. Il peut s'agir d'autres couples de molécules biologiques, que des oligonucléotides complémentaires entre eux, connus de l'homme du métier mettant en jeu des liaisons sensibles au pH, par exemple choisis dans la liste précitée, par exemple substrat/enzyme, anticorps/antigène, hormone/récepteur, etc. Cette possibilité d'accrochage spécifique permet avantageusement un choix précis du point d'accrochage pour un objet donné.

Selon une variante des deux modes de réalisation précités de la présente invention, également représenté sur la figure 1, la cible « B » peut être utilisée pour porter un objet « C », par exemple un objet à accrocher et/ou décrocher qui doit être extrait d'un échantillon, un objet à isoler, un objet à décrocher avec une temporisation, etc.

Cet objet peut être attaché à la cible « B » antérieurement, simultanément ou postérieurement à l'assemblage de « A » + « B ». Si B porte un objet « C », ce dernier est alors accroché et/ou décroché par l'intermédiaire de « B » sur A. Aucun autre phénomène électrogénéré, tel que le champ électrique, n'intervient lors de l'accrochage par voie électrochimique de l'objet « C » par exemple à la surface d'un microsystème.

Ainsi, selon l'invention, dans l'un ou l'autre des modes de réalisation, le procédé peut comprendre en outre l'étape suivante : (x) fixation d'un objet sur la cible.

Cette variante présente notamment l'avantage que, pour un couple sonde/cible déterminé, par exemple pour lequel les conditions d'accrochage/décrochage selon l'invention sont bien connues et maîtrisées, par exemple un couple sonde/cible sous la forme de brins oligonucléotidiques complémentaires ou sous une des formes présentées dans les exemples ci-dessous, il est possible d'accrocher/décrocher l'un quelconque des objets précités suivant le procédé de l'invention, pourvu qu'une liaison puisse être formée entre l'objet et la cible, et que ce couple cible/objet, puisse être accroché/décroché sur/de la sonde par le procédé de l'invention qui est mis en oeuvre.

Cette variante présente en outre l'avantage que lorsqu'un objet doit être accroché/décroché suivant le procédé de l'invention, et qu'il n'est pas possible chimiquement d'établir une interaction électrochimique directe de l'objet avec la sonde (l'objet serait alors une cible au sens de la présente invention), l'objet est en quelque sorte « porté » par la cible, et c'est cette dernière qui interagit avec la sonde pour s'accrocher ou se décrocher sur/de la zone d'accrochage. Il suffit alors de fixer l'objet sur la cible par l'un quelconque des procédés connus de l'homme du métier.

De préférence, l'objet « C » étant déterminé, on choisira un couple sonde-cible dont la reconnaissance et la liaison ne sera pas gênée et/ou empêchée par la fixation de l'objet « C » sur la cible. Inversement, pour un couple sonde-cible déterminé, on choisira l'objet « C » de manière à ce que sa fixation sur la cible ne gêne pas et/ou n'empêche pas la liaison sonde-cible.

L'objet « C » peut être par exemple choisi dans le groupe constitué d'une molécule, d'une cellule ; d'une bactérie ; de billes fonctionnalisées, par exemple de billes de latex, de verre, etc., ; d'une protéine ; d'une enzyme ; d'un anticorps, par exemple afin de reconnaître et d'immobiliser une cellule ; d'un fragment biologique; de molécules à transfecter ; de molécules d'intérêt biologique ; de principes actifs ; de molécules d'intérêt pharmacologique ; groupements chimiques, etc. L'objet « C » peut également être une molécule ou un objet tel que la cible « B ».

A titre illustratif, l'objet « C » peut être un marqueur destiné à mettre en évidence la liaison sonde-cible. Il peut s'agir par exemple d'un quelconque des marqueurs connus de l'homme du métier utilisés pour mettre en évidence des molécules chimiques ou biologiques ou des réactions de reconnaissance moléculaire entre une sonde et sa cible sur une biopuce. Il peut s'agir par exemple d'une molécule fluorescente, d'une molécule électroactive, etc. Un exemple d'un tel assemblage est donné ci-dessous.

Dans l'étape précitée d'accrochage d'un objet sur la cible, l'objet étant un marqueur, le procédé de l'invention peut comprendre en outre une étape de détection de la cible marquée. Les marqueurs précités étant connus, il n'est pas nécessaire de rappeler ici les techniques connues de l'homme du métier pour détecter un marqueur.

Dans les procédés de la présente invention, la sonde peut également porter une molécule telle qu'un marqueur, par exemple pour mettre en évidence la liaison sonde-cible, pourvu que cette molécule ne gêne pas ladite liaison. Cette molécule peut être fixée sur la sonde par les techniques habituelles de chimie, avant ou après la fonctionnalisation de la zone d'accrochage par la sonde. Par exemple, lorsque cette molécule est un marqueur, elle peut être choisie parmi une molécule chimique (par exemple la dioxygénine), un fluorophore (par exemple la fluorescéine, un « molecular beacon » et son marqueur fluorescent), une molécule active électrochimiquement (par exemple le ferrocène), une molécule active biologiquement (par exemple une enzyme), un marqueur radioactif (par exemple contenant un ou des isotope(s) du phosphore [P32]). Dans un autre exemple de réalisation de la présente invention, une biotine peut par exemple être fixée sur la sonde (la sonde porte une biotine), et la cible peut être marquée par de la streptavidine-phycoérythrine.

Selon l'invention, l'accrochage de la cible « B » par la sonde « A » peut être précédé d'une activation électrochimique de la sonde, par application d'un (de) potentiel(s) ou d'un (de) courant(s) électrochimique(s), à l'électrode de travail. Cette activation permet d'effectuer une variation locale du pH (acidification ou basification), limitée au voisinage de l'électrode de travail (électrode active) et de la zone d'accrochage. L'application de potentiel(s) ou de courant(s) électrochimique(s) adapté(s) provoque une réaction électrochimique dont les produits entraînent par exemple une hydroxylation ou une protonation instantanée et localisée de la solution en contact avec le microsystème, et ainsi, la formation de la liaison entre la sonde et la cible, et l'accrochage de cette dernière par la sonde sur la zone d'accrochage. Par exemple, les groupements électrophiles et les groupements nucléophiles de la sonde et de la cible interagissent et s'accrochent suivant A + B → A-B.

L'activation électrochimique peut être réalisée dans la (les) solution(s) servant à l'assemblage, par exemple « A » + « B-C » ou « A » + « B » + « C » ou « A-B » + « C », donc ne nécessite pas de rinçage du système, ou dans toute autre solution « post-assemblage » si celle-ci est au moins en partie aqueuse, de préférence saline, et de préférence encore tamponnée, pourvu que les liaisons en jeu ne soient pas empêchées dans la mise en oeuvre du procédé de l'invention.

Le processus de décrochage par voie électrochimique de la cible B, ou B-C si un objet « C » est accroché à la cible, implique d'une part un accrochage réversible entre la sonde « A » et la cible « B » par pont d'accrochage comportant des groupements sensibles au pH comme décrit ci-dessus, et d'autre part, une coupure de ce pont réalisée localement (à l'endroit à décrocher), suite à une variation locale et réversible du pH. Ce processus de décrochage est représenté schématiquement sur la figure 2.

L'application de potentiel(s) ou de courant(s) électrochimique(s) adapté(s) provoque une réaction électrochimique dont les produits entraînent par exemple une hydroxylation ou une protonation instantanée et localisée de la solution en contact avec le microsystème, et ainsi, la rupture de la liaison entre la sonde et la cible. Lors de l'étape de décrochage, sous l'influence de la variation de pH électrochimique, les groupements sensibles à cette variation (par exemple liaisons hydrogène, peptidique, amide, sulfonamide, ester d'acide carboxylique, ester d'acide sulfonique, silanoate substitué) se séparent suivant A-B → A + B. Cette coupure, réversible et localisée, du pont d'accrochage peut également provoquer le détachement de l'objet « C », s'il est présent, qui retourne alors en solution sous la forme B-C ou B + C. Le détachement de C dépend bien entendu de la nature chimique de la liaison qui le lie à B. L'application d'un (de) potentiel(s) ou d'un (de) courant(s) électrochimique(s) adapté(s) provoque une réaction électrochimique dont les produits entraînent une hydroxylation ou une protonation instantanée et localisée de la solution en contact avec la zone d'accrochage, par exemple du microsystème (solution d'assemblage ou post-assemblage) qui suivant les groupements en cause dans la liaison sonde-cible entraîne le décrochage de la cible par la sonde.

Aucun autre phénomène électro-généré, tel que le champ électrique, n'intervient lors de la modulation de surface (c'est-à-dire état accroché ou décroché) par voie électrochimique. C'est un des nombreux avantages de la présente invention.

Des accrochages et des décrochages consécutifs sur une même surface, réalisés par les inventeurs, ont montré que les procédés de l'invention sont reproductibles. En effet, les procédés d'accrochage et/ou de décrochage ne sont pas définitifs. Par exemple, grâce à l'existence d'une liaison réversible, telle que celles précitées, dans le pont d'assemblage « A-B », l'accrochage et le décrochage peuvent être répétés et ce, sans altération des propriétés de la surface du microsystème, comme cela est montré dans les exemples ci-dessous. Il s'agit d'un des nombreux avantages de la présente invention.

Dans le dispositif de la présente invention, les électrodes de travail et contre-électrode peuvent être, comme habituellement pour ces éléments, notamment dans les microsystèmes connus de l'homme du métier, constituées d'une matière conductrice, et de préférence d'un métal noble, ou un alliage de métal noble, par exemple Au, Pt, Pd, Ir ; ou semi-conductrice, par exemple silicium dopé ou carboné, adaptée à l'électrochimie, dans la gamme de potentiels utilisés. Il peut s'agir également d'un polymère conducteur, d'une colle conductrice, de polypyrrole, etc.

Elles peuvent être disposées au voisinage de la zone d'accrochage en utilisant les techniques habituelles de microélectronique permettant de disposer des électrodes sur des microsystèmes telles que des biopuces. Il peut s'agir par exemple de techniques de dépôt sous vide de métaux par exemple par thermo évaporation, par plasma (en anglais : « Plasma-Enhanced Chemical Vapor Deposition » (« PECVD »)) ou pulvérisation (en anglais « sputtering »), etc. Des techniques utilisables dans la présente invention sont décrites dans les documents [19] et [20] de la liste de référence annexée.

La zone d'accrochage, notamment si elle requiert un traitement particulier par exemple pour la fixation de la sonde, peut être placée au voisinage des électrodes après leur fabrication. Elle peut également être placée sur la surface du support avant leur fabrication.

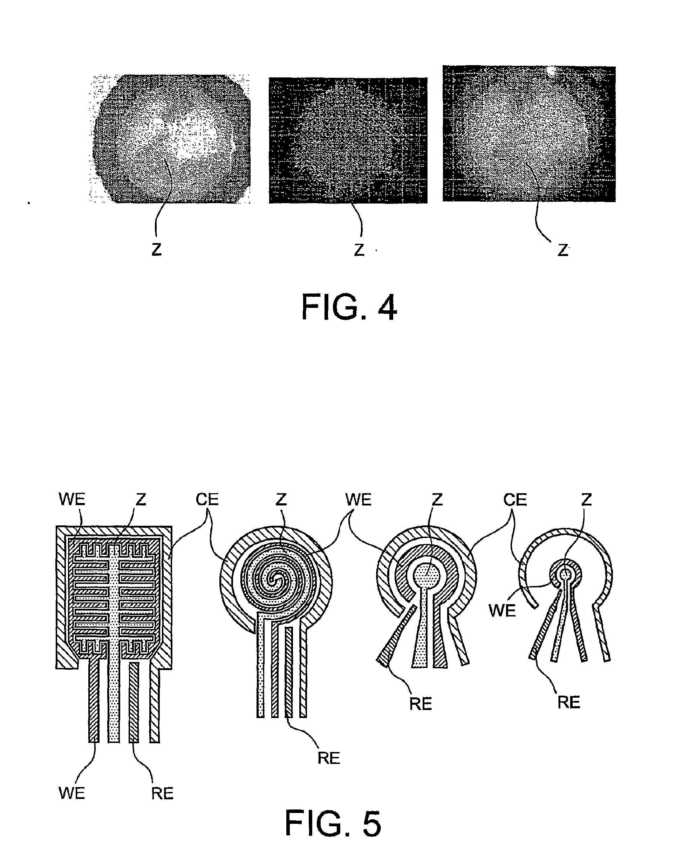

Les figures 3 et 5 annexées sont des exemples de dispositions des électrodes et de la zone d'accrochage utilisables pour constituer le dispositif de la présente invention. Bien entendu, d'autres dispositions peuvent encore être mises en oeuvre dans le cadre de l'invention à partir des informations fournies dans la présente.

Selon l'invention, de préférence, l'électrode de travail borde ou entoure la zone d'accrochage. De préférence encore, l'électrode de travail borde ou entoure la zone d'accrochage, et la contre électrode borde ou entoure ladite électrode de travail. Selon l'invention, avantageusement, l'électrode de travail, la contre électrode et la zone d'accrochage sont dans une configuration (« design » en anglais) choisie parmi une configuration de peigne inter-digité, une configuration spirale ou une configuration concentrique (voir par exemple figures 3 et 5 annexées). En effet, ces configurations sont favorables à l'application des procédés de l'invention.

La zone d'accrochage et/ou de décrochage est préférablement située au plus près de l'électrode servant à l'activation électrochimique (électrode de travail) et de préférence coplanaire à celle-ci pour assurer une meilleure action électrochimique.

Les schémas des figures 1 et 2 ont été dessinés avec une structure non coplanaire pour mieux distinguer les différents éléments du dispositif représenté. Sur la figure 3, les éléments sont coplanaires. Le positionnement de la contre-électrode peut être ou ne pas être coplanaire à la zone d'accrochage et/ou de décrochage.

Une électrode de référence (RE) peut également être jointe au dispositif, placée de manière à pouvoir mesurer le potentiel appliqué à l'électrode de travail. Pour des raisons de miniaturisation, l'électrode de référence peut être intégrée au microsystème. Il peut s'agir par exemple d'une électrode Ag/AgCl ou de toute autre électrode de référence connue de l'homme du métier et utilisable dans le dispositif de la présente invention. Sur les figures 3 et 5 annexées, sont représentées des dispositions possibles de l'électrode de référence sur des dispositifs conformes à la présente invention.

A titre d'exemple, les dimensions, dans le plan de la représentation sur ces figures, des électrodes et composants utilisés pour obtenir les dispositifs de l'invention peuvent être les suivantes pour un microsystème destiné à une application biopuce :

- zone d'accrochage (Z) : diamètre 1 à 1000μm ;
- espace entre la zone d'accrochage (Z) et l'électrode de travail (WE), et entre l'électrode de travail et la contre électrode (CE) : espace de 10 à 200μm ;
- largeur des électrodes de travail (Z) et contre électrode (CE) : 10 à 50μm ; et
- l'électrode de référence (RE) est un parallélépipède de 10 x 500 μm.

Ces dimensions ne sont en fait limitées que par les limites des dimensions qui peuvent être atteintes en microélectroniques par les techniques actuelles.

L'homme du métier peut bien entendu aisément fabriquer des dispositifs avec d'autres dimensions sur la base des informations données dans la présente et de ses connaissances générales.

Les moyens permettant d'appliquer un courant électrique ou un potentiel déterminé à l'électrode de travail sont ceux habituellement utilisés pour les microsystèmes en particulier pour des cellules électrochimiques. Ils peuvent comprendre des moyens de connexion des électrodes du dispositif à la source d'électricité permettant d'appliquer le courant électrique à l'électrode de travail. Ils peuvent comprendre en outre des moyens de commande et des moyens de mesure afin de pouvoir régler et suivre le potentiel appliqué à l'électrode de travail.

Le(s) potentiel(s) électrochimique(s) à appliquer à l'électrode de travail pour réaliser l'accrochage et/ou le décrochage dépend(ent) du type de pont d'assemblage entre la sonde et la cible (« A-B »), de la solution aqueuse électrolyte au contact du microsystème (solution de travail), de la configuration et du matériau des électrodes utilisées.

Selon l'invention, le courant peut être appliqué à l'électrode de travail de manière continue ou discontinue, croissante ou décroissante, etc. Cette application dépend notamment de la variation de pH locale que l'utilisateur désire induire. Selon l'invention, ce (s) potentiel (s) ou ce (s) courant (s) peut (peuvent) être cathodique (s), anodique(s) ou les deux.

Le courant électrique peut avantageusement être appliqué sous forme de trains de potentiels. En effet, les inventeurs ont mis en évidence qu'un train de potentiels permet avantageusement de mieux localiser la variation de pH mais aussi de la rendre réversible. Dans ce cas, les moyens permettant d'appliquer un courant électrique ou un potentiel déterminé à ladite électrode de travail comprennent avantageusement des moyens permettant d'appliquer un ou des train(s) de courant ou de potentiel(s) déterminé (s) pendant une ou des durée (s) déterminée (s) .

Ce train de potentiels ou de courants est de préférence composé au minimum d'un signal (pulse, rampe de potentiels, etc.). Le train de potentiels, ou de courants électrochimiques, appliqué peut par exemple être choisi de sorte à provoquer une variation du pH et à compenser celle-ci immédiatement, sans qu'une modification du reste de l'environnement n'ait lieu.

De manière générale, les valeurs du (des) potentiel(s) électrochimique(s) utilisées dépendent de la variation locale de pH désirée, et donc de la nature de la liaison sonde-cible en cause. En général, compte tenu du type de liaisons sonde-cible réversibles visée dans la présente invention, les valeurs de potentiels sont de préférence, sans y être limité, de -3,0 V à +4,0 V (par rapport à une électrode de référence Ag/AgCl) et de préférence de -1,8 V à -0,8 V ou entre +1,2 à +2,2 V (par rapport à une électrode de référence Ag/AgCl). Les valeurs des courants utilisés sont celles permettant d'obtenir les fenêtres de potentiels précédentes par potentiométrie.

Le potentiel ou le train de potentiels ou de courants, suivant le choix d'application du courant, est de préférence imposé pendant une durée suffisante, allant par exemple de 0,001 s à 58000 s, ou de 0,001s à 20000 s, afin de produire la variation de pH permettant l'accrochage de la cible ou son décrochage suivant le procédé appliqué.

Chaque élément du train de potentiels ou de courants possède une durée, une valeur et une forme qui lui sont spécifiques, et qui sont indépendantes des autres éléments du train de potentiels.

Pour la mise en oeuvre des procédés de l'invention, la zone d'accrochage fonctionnalisée et les électrodes sont immergés par l'échantillon aqueux. Le volume d'échantillon n'est pas déterminant car la variation de pH est précisément localisée au niveau de la zone d'accrochage. Cet échantillon est obtenu en mélangeant un prélèvement (comme défini ci-dessus), ou tout simplement la cible, avec une solution aqueuse. Les solutions aqueuses tamponnées sont préférées pour la préparation de l'échantillon, mais non indispensables à la réalisation des procédés de l'invention. En effet, l'eau suffit à l'activation électrochimique.

Le tampon permet avantageusement de limiter la diffusion des produits de la réaction électrochimique ($OH^-$ ou $H^+$) dans le reste de la solution et améliore le flux de ces produits au voisinage de l'électrode de travail et de la zone d'accrochage/décrochage. Il permet une meilleure localisation de la variation de pH, favorisant la localisation des phénomènes d'accrochage et de décrochage au niveau de la zone d'accrochage et de l'électrode de travail. Cette solution aqueuse peut être par exemple une solution de tampon phosphate, telle qu'une solution de $Na_2HPO_4$, de $NaH_2PO_4$, de $KH_2PO_4$, etc. ou d'un mélange de ces tampons. Les concentrations sont en général comprises entre 0,001 mM et 10 M, de préférence entre 1 mM et 1M.

De préférence la solution aqueuse est une solution saline. En effet, ce type de solution améliore la conduction électrique, et, par exemple dans le cas des molécules biologiques (par exemple les protéines en général) elle permet de maintenir les molécules biologiques dans leur forme active (repliement des protéines) et/ou permet la liaison sonde/cible, pour la mise en oeuvre du procédé de l'invention. Dans le cas où la sonde et/ou la cible sont des molécules biologique, la solution saline permet de mettre en oeuvre le procédé de l'invention dans une solution aqueuse de force ionique appropriée.

De préférence encore, cette solution est saline et tamponnée.

De manière générale, le choix de cette solution peut se faire notamment en fonction de la nature de la sonde et de la cible, en particulier cette solution devrait préserver les fonctions chimiques et la structure de la sonde et de la cible et permettre aussi à la sonde d'accrocher la cible. En outre elle facilite la mise en oeuvre du procédé électrochimique de la présente invention en constituant un milieu électrolyte adéquat.

Selon l'invention, la localisation de l'accrochage et/ou du décrochage de la cible peut donc être contrôlée notamment par le choix :

- du dispositif électrochimique : les dispositions particulières des électrodes à la surface du microsystème permettent la localisation de la variation de pH ;
- d'appliquer un courant ou un potentiel au choix à une ou à plusieurs cellule(s) déterminée (s) seulement, dans le cas d'une matrice ou d'un ensemble de microcellules électrochimiques selon l'invention réparties sur la surface d'un support : dans ce cas aussi, la variation de pH est localisée là où l'opérateur le désire ;
- de la solution au contact du microsystème : l'utilisation de solutions tamponnées salines est préférable ; et
- d'un procédé électrochimique approprié : par exemple, les inventeurs ont noté que l'application de trains de potentiels électrochimiques permet de mieux contrôler de la variation de pH.

La présente invention trouve de très nombreuses applications, et quelques exemples seulement sont cités ici.

La présente invention peut s'appliquer notamment à des procédés de séparation et de récupération d'objets biologiques et/ou chimiques : par exemple un tri cellulaire par accrochage sélectif, par exemple via un anticorps spécifique à ce type de cellule, à la surface du microsystème puis décrochage dans une autre solution et/ou vers une autre partie du microsystème pour la réalisation d'autres tâches telles qu'une analyse, une détection, etc.

La présente invention peut s'appliquer aussi à des fonctions de concentration d'objets biologiques et/ou chimiques : par exemple, accrochage des objets présents dans une solution circulant au-dessus de la surface d'accrochage puis décrochage dans une autre solution de volume inférieur pour faciliter la réalisation d'autres tâches telles qu'une analyse, une détection, etc.

La présente invention peut s'appliquer aussi à la réalisation de réactions biologiques et/ou chimiques nécessitant une temporisation par exemple : déprotection chimique, criblage de molécules d'intérêt pharmacologique, etc.

La présente invention peut s'appliquer aussi à des études fondamentales portant sur les propriétés physico-chimiques, thermiques, physiques, surfaciques, chimiques, ou sur le changement de fonctionnalité des objets, avant, pendant et après accrochage, par exemple : étude énergétique des mécanismes de la fixation cellulaire.

Ainsi, le dispositif de l'invention peut être utilisé par exemple dans un procédé destiné à purifier ou extraire une cible « B » ou un objet « C » fixé sur une cible « B-C » ; à concentrer une cible « B » ou un objet « C » fixé sur une cible « B-C » ; à cribler des cibles ou des objets fixés sur une cible ou à détecter une cible « B » ou un objet fixé sur une cible « B-C ». La cible « B » et l'objet « C » ont été définis ci-dessus.

La présente invention trouve par exemple aussi une application dans un procédé de purification d'une cible « B » ou d'un objet « C », ledit procédé de purification comprenant par exemple la mise en oeuvre du procédé d'accrochage « actif » ou « passif » selon l'invention, avec ladite cible « B » ou ledit objet « C » lié à une cible (ledit objet étant lié à ladite cible avant ou après l'accrochage de la cible par la sonde) (« B-C ») en utilisant un dispositif approprié selon l'invention ; un rinçage de ladite zone d'accrochage au moyen de ladite solution aqueuse ou d'une autre solution préservant la liaison sonde-cible (« A-B ») ou sonde-cible-objet (« A-B-C »); le décrochage de l'objet « B » ou cible-objet (« B-C ») soit en interrompant l'application du courant électrique à l'électrode de travail, soit en appliquant un courant électrique ou un potentiel différent au niveau de ladite zone d'accrochage de façon à modifier localement le pH de la solution de travail afin que la cible ou cible-objet se décroche de la sonde, soit en rinçant le dispositif au moyen d'une solution appropriée pour décrocher la cible ; et éventuellement le rinçage de la zone d'accrochage de manière à récupérer la cible ou cible-objet décrochée. La cible « B » et l'objet « C » ont été définis ci-dessus.

Ce procédé permet également de concentrer une cible « B » ou un objet lié à une cible « B-C », par exemple en utilisant dans le procédé de purification précité un volume de rinçage de la cible ou de la cible-objet décrochée plus petit que le volume initial de l'échantillon. La cible « B » et l'objet « C » ont été définis ci-dessus.

La présente invention trouve donc de très nombreuses applications pour la fabrication de nouvelles générations de biopuces, ou laboratoire sur puce (« lab-on-chip » en anglais). Il s'agit par exemple de microsystèmes comportant diverses étapes de travail, réalisées dans un ou plusieurs compartiments, et ce de façon consécutive. Les fonctions d'accrochage ou de décrochage peuvent utilisées comme une partie d'une ou plusieurs de ces étapes, ou comme une étape à part entière. L'accrochage et/ou le décrochage pourraient permettre de réaliser divers types de fonctions, telles que :

- des fonctions de séparation et de récupération d'objets biologiques et/ou chimiques : on peut imaginer un tri cellulaire par accrochage sélectif, par exemple via un anticorps spécifique à ce type de cellule, à la surface du microsystème puis décrochage dans une autre solution et/ou vers une autre partie du microsystème pour la réalisation d'autres tâches (analyse, détection, etc.) ;

- des fonctions de concentration d'objets biologiques et/ou chimiques : par exemple, accrochage des objets présents dans une solution circulant au dessus de la surface d'accrochage puis décrochage dans une autre solution de volume inférieur pour faciliter la réalisation d'autres tâches, par exemple d'analyse, de détection, etc. ;

- la réalisation de réactions biologiques et/ou chimiques nécessitant une temporisation, par exemple pour un criblage de molécules d'intérêt pharmacologique, une déprotection chimique, etc. ;

- des études fondamentales portant sur les propriétés physico-chimiques, thermiques, physiques, surfaciques, chimiques, ou sur le changement de fonctionnalité des objets, avant, pendant et après accrochage, par exemple des études énergétiques des mécanismes de la fixation cellulaire.

D'autres caractéristiques, avantages, et applications potentielles apparaîtront encore à la lecture des exemples qui suivent donnés à titre d'illustration en référence aux figures annexées.

**Brève description des figures**

- La figure 1 représente schématiquement une stratégie d'accrochage d'élément(s) biologique(s) et/ou chimique(s) par voie électrochimique selon le procédé de l'invention : à gauche : les différents éléments sont mis en présence (sonde « A », cible « B » et objet « C ») ; au centre : activation par voie électrochimique et variation locale du pH (indiquée par la flèche et les petits signes positifs) favorisant l'assemblage ; à droite : l'objet « C » est accroché par l'intermédiaire de la cible sur la sonde.

- La figure 2 représente schématiquement une stratégie de décrochage d'élément(s) biologique(s) et/ou chimique(s) par voie électrochimique selon le procédé de l'invention : à gauche : assemblage de la cible « B » et de l'objet « C » sur la sonde « A » ; au centre : activation par voie électrochimique au moyen de l'électrode de travail « WE » et variation locale du pH (indiquée par la flèche et les petits éclairs) pour le décrochage ; à droite : l'objet « C » est décroché avec « B » (« B-C ») .

- La figure 3 représente schématiquement deux exemples de configuration de dispositif à trois ou quatre électrodes (respectivement à gauche et à droite).

- La figure 4 représente trois photographies d'une zone d'accrochage et de décrochage sur laquelle des objets fluorescents ont été accrochés selon le procédé de la présente invention : immédiatement après accrochage (photo de gauche), après décrochage électrochimique (photo du milieu) et après un nouvel accrochage (photo de droite).

- La figure 5 représente schématiquement des exemples de configurations de dispositifs conformes à la présente invention comprenant : une zone d'accrochage (Z), une électrode de travail (WE), une contre-électrode (CE) et une électrode de référence (RE) respectivement de gauche à droite : sous forme inter-digitée, spirale et concentrique, constituant des microcellules électrochimiques adaptées à l'accrochage au décrochage d'objets selon l'invention. Noir = zone d'accrochage et/ou de décrochage, gris foncé = électrode de travail, gris clair = contre électrode, hachuré = électrode de référence.

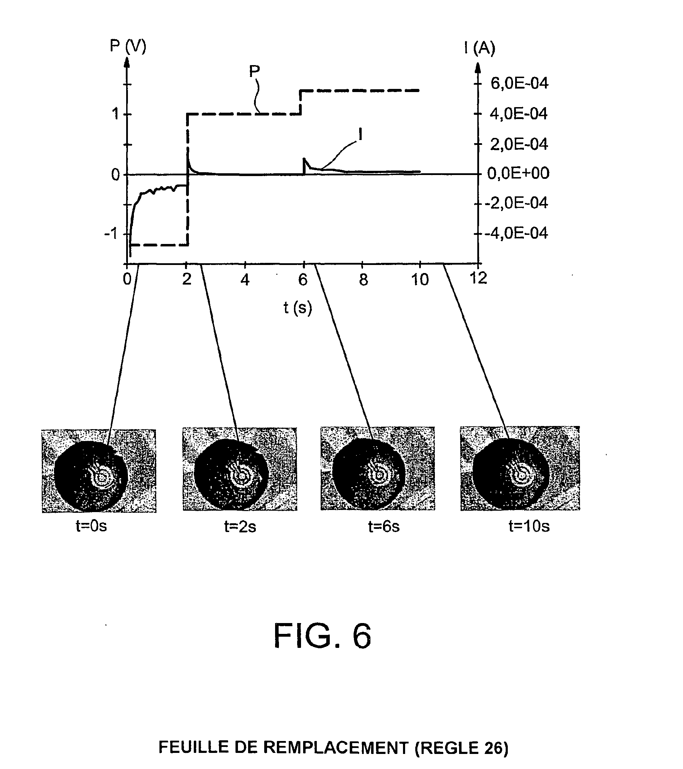

- La figure 6 représente trois photographies d'une microcellule électrochimique selon la présente invention dans trois états différents : au repos (image de gauche), pendant l'activation électrochimique (image du milieu) et après correction électrochimique de la variation de pH (image de droite). La variation de pH est mise en évidence à l'aide d'un indicateur coloré, ici le rouge de crésol. Ces trois photographies sont mises en relation avec un graphique montrant l'application du potentiel (P) (en V) et du courant (I) obtenu (en A) à l'électrode de travail en fonction du temps (t) (en s).

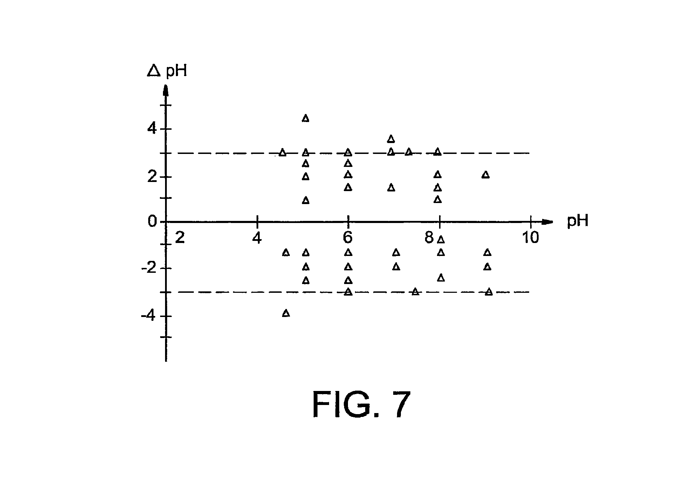

- La figure 7 est un graphe représentant la variation de pH ($\mu$pH) obtenue par voie électrochimique au voisinage de l'électrode de travail et de la zone d'accrochage/décrochage d'un dispositif selon l'invention en fonction du pH (pH initial) du milieu dans lequel le dispositif est immergé (solution tampon) .

## EXEMPLES

### Exemple 1 : fabrication d'un dispositif selon l'invention

Dans cet exemple, le dispositif de l'invention est fabriqué sur un support de silice suivant le protocole exposé dans le document [15] dans la liste de référence annexée. La fabrication du dispositif comprend les étapes suivantes :

Sur un substrat de silicium :

- oxydation de la surface du substrat sur une épaisseur de 1 $\mu$m ;
- dépôt d'une couche de métal (Pt ou Au) sur une couche d'accrochage de Ti par une technique par évaporation ;
- structuration des électrodes par photolithographie et gravure sèche ;
- passivation des pistes par dépôt d'oxyde de silicium suivi d'une ouverture (photo puis gravure de l'oxyde) des électrodes de mesure ;
- dépôt électrochimique d'argent sur les électrodes de références ; et
- étude de la chloration chimique des électrodes.

Les dispositifs fabriqués sont ensuite connectés à un potentiostat (AutoLab PGstat 100 de la société Ecochemie) afin de pouvoir mesurer et contrôler le potentiel et/ou le courant appliqué. La configuration utilisée est une configuration classique pour des cellules électrochimiques, c'est-à-dire une configuration à trois électrodes : une électrode de travail, une contre-électrode, et une électrode de référence, cette dernière étant optionnelle.

### Exemple 2 : exemple de configurations de dispositifs selon l'invention (microcellules électrochimiques)

Différents microsystèmes électrochimiques dont la configuration est adaptée à l'accrochage et au décrochage par voie électrochimique selon l'invention ont été réalisés suivant le protocole de l'exemple 1.

Ces dispositifs sont représentés schématiquement sur la figure 5 annexée. Sur cette figure, « CE », « WE », « Z » et « RE » représentent respectivement la contre électrode, l'électrode de travail, la zone d'accrochage et/ou de décrochage, et l'électrode de référence.

Les dimensions, dans le plan de la représentation, des électrodes et composants utilisés pour les dispositifs fabriqués dans cet exemple sont données en exemple pour le dispositif représenté sur le troisième schéma de la figure 5 en commençant par la gauche :

- zone d'accrochage (Z) : diamètre 300$\mu$m ;
- espace entre la zone d'accrochage (Z) et l'électrode de travail (WE), et entre l'électrode de travail et la contre électrode (CE) : espace constant d'environ 70$\mu$m ;
- largeur des électrodes de travail (WE) et contre électrode (CE) : 130$\mu$m ; et
- l'électrode de référence (RE) est un parallélépipède de 50 x 200 $\mu$m.

  Les autres dispositifs sur cette figure sont représentés sensiblement à la même échelle.

### Exemple 3 : préparation de la zone d'accrochage et fonctionnalisation de cette zone par la sonde

Différentes préparations de différentes zones ont été réalisées dans cet exemple dans différents essais, par différentes techniques :

A) Préparation d'une zone d'accrochage par électrogreffage de polymères conducteurs et fonctionnalisation par des oligonucléotides

Dans cet exemple, la zone d'accrochage est préparée par électrogreffage d'une couche de polymères constituée de pyrrole et d'oligonucléotides-pyrrole, selon les conditions et le protocole décrit dans le document référencé [18].

L'électrocopolymérisation du polymère des deux monomères entraîne l'immobilisation du brin oligonucléotide (la sonde) par sa terminaison pyrrole.

B) Préparation d'une zone d'accrochage par silanisation et immobilisation de diverses fonctions chimiques

Les protocoles suivants sont donnés pour un greffage de silane portant différentes fonctions. Dans ces protocoles, la sonde est la fonction portée par le silane.

(i) Fonction époxyde

On utilise le protocole de silanisation décrit dans la demande WO-A-02/051856 :

- réhydratation de la surface du microsystème (formation de fonctions silanol, SiOH) dans une solution contenant 7g NaOH + 21 ml d'eau distillée + 28ml éthanol pendant 2 heures sous agitation à température ambiante ;
- lavages abondants à l'eau désionisée ;
- séchage à 80°C pendant 15 minutes ;
- réaction dans un mélange toluène 30ml + triéthylamine 0,9ml + 5,6-époxyhexyltriéthoxysilane 36μl pendant 16 heures à 80°C ;
- rinçage à l'acétone; et
- réticulation 3 heures à 110°C.

(ii) Fonction aldéhyde

On utilise le protocole de silanisation décrit dans la demande WO-A-02/051856 :

- réhydratation de la surface du microsystème (SiOH) dans une solution contenant 7g NaOH + 21 ml d'eau distillée + 28ml éthanol pendant 2 heures sous agitation à température ambiante ;
- lavages abondants à l'eau désionisée ;
- séchage à 80°C pendant 15 minutes ;
- réaction dans un mélange toluène 30ml + triéthylamine 0,9ml + 5,6-époxyhexyltriéthoxysilane 36μl pendant 16 heures à 80°C ;
- rinçage à l'acétone ;
- réticulation 3 heures à 110°C ;
- hydrolyse acide dans HCl 0,2N pendant 3 heures à température ambiante ;
- rinçages à l'eau distillée ; et
- oxydation des fonctions diols de surface en fonctions aldéhydes pendant 1 heure à température ambiante dans une solution de NaIO$_4$ (660mg de NaIO$_4$, 30ml eau désionisée).

(iii) Fonction halogénure

On utilise le protocole suivant :

- réhydratation de la surface du microsystème (SiOH) dans une solution contenant 7g NaOH + 21 ml d'eau distillée + 28ml éthanol pendant 2 heures sous agitation à température ambiante ;
- lavages abondants à l'eau désionisée ;
- séchage à 80°C pendant 15 minutes ;
- réaction dans un mélange toluène 20ml + di-isopropyléthylamine 0,6ml + ((p-chlorométhyl) phényléthyl) triméthoxysilane 50μl pendant 24 heures à température ambiante ;
- rinçage à l'éthanol ; et
- réticulation 3 heures à 110°C.

C) Préparation d'une zone d'accrochage par adsorption de polymères, de protéines ou de thiols porteurs de fonctions chimiques

Dans tous les cas, il s'agit d'immerger l'électrode ou la surface constituant la zone d'accrochage dans la solution faite du solvant adapté à chaque type de molécule :

- solution aqueuse pour les protéines,
- éthanol pour les thiols,

pendant une durée suffisante (à température ambiante) :

- 1 heure pour les protéines,
- 24 heures pour les thiols, sous argon.

Les procédés utilisés sont décrits dans les documents référencés [8] à [12] de la liste de référence annexée. Dans tous ces procédés ci-dessus, on peut remplacer l'eau distillée par de l'eau désionisée.

Dans ces cas, la sonde est soit une fonction portée par les thiols greffés, soit la protéine ou le polymère immobilisé, par exemple à la surface du microsystème, pour former la zone d'accrochage.

**Exemple 4 : exemple d'accrochage « passif » et de décrochage « actif » réversible par voie électrochimique selon la présente invention**

Dans cet exemple, l'accrochage utilisé est le suivant :

- La sonde (« A ») est un brin d'oligonucléotide immobilisé par du polypyrrole sur une zone d'accrochage d'un dispositif selon l'invention réalisé comme dans les exemples 1 et 2 ;

- La cible « B » est un oligonucléotide complémentaire de la sonde « A », et portant une biotine ;
- Une autre molécule, appelée « C », objet à ancrer via la cible sur la zone d'accrochage, est un marqueur : une streptavidine-phycoérythrine (conjugué fluorescent).

Le protocole d'accrochage de la sonde est celui décrit dans le document référencé [18]. Les oligonucléotides ont été modifiés pour qu'ils comportent un groupement pyrrole. Ces oligonucléotides modifiés sont ensuite immobilisés par électrogreffage sur la zone d'accrochage.

Les cibles complémentaires biotinylées ($0,1\mu M$) ont été hybridées 15 minutes à 50°C et l'objet (streptavidine phycoérythrine, solution commerciale diluée 10 fois) a ensuite été immobilisé sur la zone d'accrochage par affinité chimique entre la biotine et la streptavidine pendant 5 minutes à température ambiante.

Des étapes de rinçage sont effectuées avec un tampon phosphate salin (NaCl = 27mM, KCl = 138mM) + 0,3% de tween.

La présence de fluorescence est caractéristique de l'état accroché de l'objet « C ». Cette fluorescence est observée sous microscope de fluorescence (Provis (marque de commerce)).

Pour l'activation électrochimique : le microsystème est connecté à un potentiostat (AutoLab PGstat100, de la société Ecochemie) et par chronoampérométrie : un potentiel de V = -1,2V, par rapport à une électrode de référence Ag/AgCl, est appliqué à l'électrode de travail du microsystème pendant 2 secondes.

L'état ancré de la cible par la sonde est confirmé par observation du signal de fluorescence comme cela apparaît sur la figure 4 annexée, image de gauche. L'état décroché est vérifié lorsque le signal de fluorescence disparaît comme cela est visible sur la figure 4 annexée, image centrale.

Ce processus électrochimique n'affecte pas la réversibilité de l'accrochage, car un nouvel accrochage d'objets fluorescents a été effectué dans les mêmes conditions et s'est accompagné de l'observation d'un nouveau signal de fluorescence visible sur la figure 4, image de droite.

Des accrochages et des décrochages successifs sur une même zone d'accrochage et de décrochage ont en outre montré que le processus peut être répété sans altération de la fonctionnalité « A » de cette zone d'accrochage et de décrochage.

**Exemple 5 : exemple de train de potentiels électrochimiques permettant de gérer et de localiser la variation électro-commandée du pH dans le procédé de l'invention**

Le choix de la configuration des électrodes, de la solution support mais aussi le choix d'un train de potentiels adapté, permet de contrôler la localisation du processus de décrochage.

Par exemple, des tests colorimétriques et électrochimiques réalisés à l'aide de microélectrodes concentriques comme celles fabriquées dans l'exemple 2, ont mis en évidence la possibilité de localiser de manière précise au voisinage de l'électrode de travail (électrode active) le phénomène de variation électrochimique du pH.

Par exemple, une goutte de solution aqueuse contenant un indicateur coloré par exemple choisi parmi le Bleu de Bromocrésol, le Vert de Bromocrésol, le Bleu de Thymol, la Phénolphtaléine et le Rouge de Chlorophénol, le Rouge de Crésol ou le Tropéoline O, vire seulement au-dessus de l'électrode active et de la zone d'accrochage et/ou de décrochage et disparaît rapidement lorsque la suite du train de potentiels électrochimiques est appliqué comme cela est visible sur la figure 6 annexée. Le virage des indicateurs précités se situe entre 0,2 et 13 unité pH.

Mode opératoire : dans différents essais, des microcellules électrochimiques fabriquées comme dans les exemples 1 et 2 ont été recouvertes de solutions tamponnées de phosphates ($KH_2PO_4$ et $Na_2HPO_4$) de différents pH : 4,6 ; 5,1 ; 6,0 ; 7,0 ; 7,4 ; 8,0 et 9 ; et contenant quelques % de divers indicateurs colorés : Phénolphtaléine, Rouge de Crésol, Bleu de Thymol, Bleu de Bromothymol, Vert de Bromocrésol, Jaune de Méthyle et Rouge de Chlorophénol ; dont les zones sont comprises entre pH 0,2 et 10.

Ces microsystèmes sont ensuite connectés à un potentiostat et des potentiels de -0,8V à -1,4V et de +0,8V à +2,0V sont appliqués pendant 2 à 10s.

Les variations de couleurs sont observées sous lumière blanche à l'aide d'un banc optique composé d'une binoculaire, d'une caméra CDD couleur et d'un système d'acquisition d'images.

Les variations de pH obtenue sur l'électrode de travail (WE) et la zone d'accrochage (Z) vont jusqu'à +/- 3 unités de pH comme cela est représenté sur le graphique de la figure 7 annexée.

L'application de ce train de potentiels permet une compensation immédiate de la variation de pH.

Des photographies de la localisation de la variation de pH mise en évidence par un indicateur coloré, ici le rouge de Crésol, ont été réalisées. Elles mettent en évidence la variation locale du pH par voie électrochimique, sur une microcellule concentrique.

La variation est bien confinée au dessus de l'électrode de travail et de la zone fonctionnalisée centrale (surface d'accrochage et de décrochage) conformément à l'attente des inventeurs, et grâce à la mise en oeuvre de la présente invention..

La figure 6 annexée est une représentation de train de potentiels à 3 pulses et de photographies correspondantes du dispositif de l'invention (avant et après chaque pulse).

Sur cette figure, Graphe I = f(t) (I en A et t, le temps, en s) : courant obtenu suite à l'application d'un train de potentiels électrochimique permettant la variation de pH et sa compensation.

Ces images illustrent la compensation de la variation de pH d'une goutte de solution tamponnée (PBS pH = 7,4) + rouge de Crésol. La couleur rouge est indicative d'un pH supérieur à 8,8. Le potentiel (en V) est également indiqué.

## Exemple 6 : exemple d'accrochage par voie électrochimique

Suivant le procédé de silanisation décrit dans la demande de brevet WO-A-02/051856, des fonctions époxydes sont greffées à la surface de microsystèmes selon l'invention pour former des zones d'accrochage.

Ces dernières sont ensuite mises en présence d'une solution de tampon phosphate + 10% de glycérol contenant des oligonucléotides (ODN) porteurs d'une fonction amine terminale en 5' (ODN-NH$_2$). Cette solution forme la solution de travail au sens de la présente invention.

Pour l'accrochage de ODN-NH$_2$ sur la zone d'accrochage : La réaction entre les fonctions époxyde et amine est activée par hydroxylation électrochimique suivant le procédé de l'invention : application d'un potentiel de -1,2V, par rapport à une électrode de référence Ag/AgCl, pendant 1200s à l'électrode de travail.

Ce protocole entraîne une réaction entre la fonction amine de l'ODN-NH$_2$ et les fonctions époxyde de la zone d'accrochage. Les oligonucléotides sont alors accrochés à la zone d'accrochage. La détection de cet accrochage est faite par mesure de fluorescence : un ODN cible marqué d'un fluorophore (streptavidine-CY3) est hybridé et le microsystème est observé à l'aide d'un microscope de fluorescence.

L'accrochage des oligonucléotides par voie électrochimique a été observé en image en lumière blanche et sous lumière fluorescente.

## Exemple 7 : Décrochage de cellules immobilisées à la surface d'un microsystème

Une matrice protéique a été greffée par adsorption pendant 1 heure à température ambiante sur des dispositifs selon l'invention pour former des zones d'accrochage selon l'invention. Ces dernières ont été immergées dans un milieu de culture cellulaire contenant des cellules HELA (environ 2,76.10$^6$c/ml).

Après 2 heures d'incubation, quelques cellules présentaient une forme étalée, caractéristique de leur immobilisation sur une surface.

Un décrochage électrochimique suivant le procédé de l'invention a été réalisé dans une goutte de PBS (pH = 7,4 [NaCl] = 2,7 mM et [KCl] = 138 mM) en modulation de pH pour protéger les cellules. L'activation électrochimique est similaire à celle présentée à l'exemple 5.

On constate que leur forme devient arrondie, ce qui signifie qu'elle se détache progressivement de la surface à laquelle celles-ci adhéraient.

Ces résultats expérimentaux confirment l'utilisation de la présente invention pour l'accrochage et/ou le décrochage d'objet s biologiques sur un microsystème électrochimique selon la présente invention.

## Exemple 8 : exemple d'accrochage et décrochage d'une biotine par voie électrochimique

Suivant le procédé de fonctionnalisation par silanisation décrit dans l'exemple 3 B) (ii), des fonctions aldéhydes sont accrochées à la surface et sont ensuite mises en présence d'une solution de tampon phosphate salin (« PBS » pH = 7,4, NaCl = 27mM, KCl = 138mM) contenant des biotine-amine ([20mM]) (molécule disponible chez Molecular Probes).

La réaction entre les fonctions aldéhyde et amine est activée par hydroxylation électrochimique (-1,2V, par rapport à une électrode de référence Ag/AgCl, pendant 7200s, AutoLab PGstat100, Ecochemie) dans une chambre humide et entraîne la création d'une liaison imine. L'ensemble biotine-amine est alors accroché à la surface.

Pour vérifier l'accrochage, un fluorophore (streptavidine-phycoérythrine) est couplé à la biotine (mise en présence pendant 5 minutes puis rinçage avec une solution PBS). La détection de l'état accroché est faite à l'aide d'un microscope de fluorescence.

Le clivage de la fonction imine est ensuite réalisé par protonation électrochimique locale de la solution en contact avec le microsystème. Un potentiel de +1,6V par rapport à une électrode de référence Ag/AgCl est appliqué (AutoLab PGstat100, Ecochemie) pendant 7200s et, après rinçage avec une solution tampon, le microsystème est observé sous un microscope de fluorescence.

La disparition du signal est indicatrice du décrochage.

Une autre réaction d'accrochage est effectuée qui vérifie la complète réversibilité et prouve que la disparition du signal est seulement due au décrochage de la cible, c'est-à-dire de la biotine.

## Exemple 9 : exemple d'accrochage et décrochage d'une cible par voie électrochimique

Dans cet exemple, on prépare une zone d'accrochage capable d'accrocher une cible « B » capable de « porter » un objet « C ».

La zone d'accrochage est fonctionnalisée par une fonction aldéhyde (sonde « A »), la cible « B » est le dihydrochlorure 2-hydrazinopyridine porteur d'une fonction hydrazine.

Suivant le procédé de fonctionnalisation par silanisation décrit dans l'exemple 3 B)(ii), des fonctions aldéhyde sont accrochées à la surface et sont ensuite mises en présence d'une solution de tampon phosphate contenant un dihydrochlorure 2-hydrazinopyridine porteur d'une fonction hydrazine ([25mg/ml]).

La formation d'une liaison hydrazone, entre les fonctions aldéhyde et hydrazine, est permise (déprotection de hydrochlorure d'hydrazine en milieu alcalin) par hydroxylation électrochimique (-1,2V, par rapport à une électrode de référence Ag/AgCl, pendant 58000s, Auto-Lab PGstat100, Ecochemie) dans une chambre humide.

L'accrochage est vérifié par Infrarouge multi-réflexions, où un pic caractéristique du groupement pyridine est détecté.

Le clivage de la fonction hydrazone est ensuite catalysé par protonation électrochimique locale de la solution en contact avec le microsystème Un potentiel de +1,6V, par rapport à une électrode de référence Ag/AgCl, est appliqué (AutoLab PGstat100, Ecochemie) pendant 22000s et, après rinçage avec une solution tampon, le microsystème est observé.

La disparition du pic est indicatrice du décrochage.

## Exemple 10 : exemple d'accrochage et décrochage d'un objet par voie électrochimique

On utilise la zone de fonctionnalisation et la cible de l'exemple 9.

Sur la cible comportant une fonction ester activée et une fonction hydrazine protégée (par exemple hydrochlorure succinimidyl hydraziniumnicotinate), on greffe une protéine (objet « C »), puis la fonction hydrazine est déprotégée afin de pouvoir être accrochée.

L'ensemble cible-objet est « accrochable » et « décrochable » sur la zone de fonctionalisation par la cible qui sert ici d'intermédiaire (« linker » en anglais) suivant le procédé de la présente invention.

## Exemple 11 : exemple d'accrochage et décrochage d'un anticorps

Cet exemple montre une mise en oeuvre de la présente invention avec une molécule biologique.

Le protocole d'accrochage de la sonde est celui décrit dans le document référencé [18]. Il s'agit d'immobiliser par électrogreffage des oligonucléotides modifiés (comportant un groupement pyrrole). Les cibles complémentaires biotinylées ($0,1\mu M$) sont hybridées 15 minutes à 50°C.

Un empilement protéique (avidine / protéine A biotinylée/ anticorps) est alors réalisé. Les différentes étapes de l'empilement sont réalisées, soit in-situ, soit en solution (PBS) et l'empilement peut être réalisé élément par élément ou en mélange global. Dans tous les cas, chaque étape s'effectue par mise en présence des éléments pendant 1 heure et 30 minutes sous agitation et à température ambiante. Les concentrations utilisées sont de 1mg/ml en Avidine et protéine A et de 20mg/ml d'anticorps (AC anti E. Coli). Les étapes de rinçage sont effectuées avec un tampon phosphate salin (PBS pH = 7,4, NaCl = 27mM, KCl = 138mM) + 0,3% de tween. Pour vérifier l'accrochage, un anticorps marqué à la fluorescéine est utilisé. L'observation sous microscope d'un signal de fluorescence est caractéristique de l'état accroché de l'objet.

Décrochage électrochimique : le microsystème est connecté à un potentiostat (AutoLab PGstat100, Ecochemie) et par chrono-ampérométrie un potentiel de V = -1,2V, par rapport à une électrode de référence Ag/AgCl, est appliqué au microsystème pendant 4 s.

Après rinçage avec une solution PBS + 0,3% de tween, le microsystème est observé sous un microscope de fluorescence. La disparition du signal est indicatrice du décrochage.

Une autre réaction d'accrochage effectuée vérifie la complète réversibilité prouve que la disparition du signal est uniquement due au décrochage de la cible, c'est-à-dire de l'anticorps et le reste de l'empilement protéique.

## Exemple 12 : exemple d'accrochage d'un ferrocène

Cet exemple montre une mise en oeuvre de la présente invention avec une molécule chimique.

La fonctionnalisation est réalisée par greffage d'un thiol acide : immersion de la surface du microsystème dans une solution de 1mM d'acide 11-mercapto-undecanoïque dans de l'éthanol pendant 24 heures sous Argon, rinçage dans un bain à ultrasons dans de l'éthanol pendant 10 minutes. Puis une fonction ester activée est synthétisée par réaction entre la fonction acide et le N-hydroxysuccinimide. L'acide est mis en présence de N-hydroxysuccinimide (4mM) + du N,N'-dicyclohexylcarbodiimide (4mM) dans du chloroforme pendant 2 heures à température ambiante, suivant le protocole de la référence [21].

La surface du microsystème présente alors des fonctions ester activées. Ces dernières sont ensuite mises en présence d'une solution de tampon phosphate (pH 7,4) contenant des ferrocènes-amine ([20mM], synthétisés selon le protocole décrit dans la référence [22].

Une activation électrochimique catalyse la formation d'une liaison amide. L'accrochage par hydroxylation a lieu dans les conditions suivantes : - 1,4V par rapport à une électrode de référence Ag/AgCl pendant 22000s (AutoLab PGstat100, Ecochemie). Les ferrocènes sont alors accrochés à la surface. La détection est faite par électrochimie (voltamétrie cyclique, balayage de 0V à 0,5V par rapport à une électrode de référence Ag/AgCl, à 50mV/s). L'observation d'un pic d'oxydation dans cette gamme de potentiels est caractéristique de la présence de ferrocène.

## Exemple 13 : premier exemple d'accrochage et décrochage d'une bactérie

Cet exemple montre une mise en oeuvre de la présente invention avec un objet biologique.

Le protocole d'accrochage de la sonde est celui décrit dans le document référencé [18]. Il s'agit d'immobiliser par électrogreffage des oligonucléotides modifiés (comportant un groupement pyrrole). Les cibles complémentaires biotinylées (0,1$\mu$M) ont été hybridées 15 minutes à 50°C. Un empilement protéique (avidine / protéine A biotinylée/ anticorps + bactérie) est alors réalisé.

Les différentes étapes de l'empilement sont réalisées, soit in-situ, soit en solution et l'empilement peut être réalisé élément par élément ou en mélange global. Dans tous les cas, chaque étape s'effectue par mise en présence des éléments pendant 1 heure et 30 minutes sous agitation et à température ambiante.

Les concentrations utilisées sont de 1mg/ml en Avidine et protéine A et 20mg/ml d'anticorps (AC anti E. Coli). La concentration de la suspension d'E.Coli (DH5$\mu$) est celle d'une culture d'une nuit (ensemencement de 2ml de milieu de culture LB, Gibco-BRL) concentrée 15 fois. Les étapes de rinçage sont effectuées avec un tampon phosphate salin (PBS pH = 7,4, NaCl = 27mM, KCl = 138mM).

La présence de bactéries est observée sous microscope en lumière blanche.

Décrochage électrochimique : le microsystème est connecté à un potentiostat (AutoLab PGstat100, Ecochemie) et par chrono-ampérométrie un potentiel de V = -1,2V, par rapport à une électrode de référence, Ag/AgCl est appliqué au microsystème pendant 4 secondes.

Il a été vérifié au préalable que ces bactéries supportent ces conditions électrochimiques. Une goutte de suspension de bactéries marquées à l'Acrydine (indicateur de l'état vivant ou mort) a été déposée sur le microsystème des conditions électrochimiques identiques ont été appliquées et les bactéries ont été observées sous lumière fluorescente.

Celles-ci sont toujours vivantes après application du pH électrochimique.

Après rinçage avec une solution PBS, le microsystème est observé sous un microscope en lumière blanche pour vérifier l'absence de bactéries.

**Exemple 14 : deuxième exemple d'accrochage et décrochage d'une bactérie**

Le protocole appliqué dans cet exemple est le même que dans l'exemple 13, sauf que la 1$^{ere}$ partie du protocole, jusqu'à l'empilement, est réalisée suivant l'exemple 8 ci-dessus.

Des résultats équivalents à ceux de l'exemple 13 sont observés.

**Exemple 15 : exemple d'accrochage d'un aminoglycanne**

Cet exemple montre une mise en oeuvre de la présente invention avec une molécule biologique d'intérêt pharmacologique.

Des fonctions halogénures (Cl) sont greffées à la surface des microsystèmes suivant le protocole décrit à l'exemple 3 B) (iii) .

Les halogénures (Cl) sont mis en présence d'une solution de glucosamine (100mM). La réaction de substitution des fonctions halogénures (Cl) par des fonctions amines est activée par hydroxylation électrochimique (-1,2V, par rapport à une électrode de référence Ag/AgCl, pendant 7200s, AutoLab PGstat100, Ecochemie).

La présence de sucre est vérifiée par Infrarouge multi-réflections, où des pics caractéristiques des groupements particuliers des sucres, qui sont des liaisons d'un hétérocycle (par exemple, les liaison C-C, C-O, C-H, O-C-OH, C-N, O-H, N-H, etc.) sont détectés.

**BIBLIOGRAPHIE**

[1] E. Katz & al., pH switched electrochemistry of pyrroloquinoline quinone at Au electrodes modified by functionalized monolayers, Journal of Electroanalytical Chemistry, 1996, 408, 107-112.

[2] M.N. Yousaf & M. Mrksich, Dynamic substrates : modulating the behaviours of attached cells, New Technologies for life sciences : A trends guide (Elsevier), Dec. 2000, 28-35.

[3] J.B. Oster (Combimatric Corporation), Overlaying electrodes for electrochemical microarrays, patent number WO 02/090963 A1, November 14, 2002.

[4] V. Chechik & al., Reactions and reactivity in SAMs, Advanced Materials, 2000, 12(16), 1161.

[5] E. Kaganer & al., Surface Plasmon Resonance Characterization of Photoswitchable Antigen-Antibody Interactions, Langmuir, 1999, 15(11), 3920-3923.

[6] W.E. Hennink (Universiteit van Utrecht (NL) et Stichting voor de Technische Weterschappen (NL)), Polymères LCST, brevet numéro EP 1 072 617 A1, 31 janvier 2001.

[7] M. Yamato et al., Novel patterned cell coculture utilizing thermally responsive grafted polymer surfaces, Journal of Biomedical Materials Research, 2001, 55(1), 137-140.

[8] D.J. Revell & al., SA carbohydrate Ms : formation and surface selective molecular recognition, Langmuir, 1998, 14, 4517-4524.

[9] J. Spinke & al., Molecular recognition at SAM : optimisation of surface fictionalisation, Journal of Chemical Physics, 1993, 99(9), 7012--019.

[10] C.D. Tidwell & al., Endothelial cell growth and protein adsorption on terminally functionalized, SAMs of alkanethiolates on gold, Langmuir, 1997, 13, 3404-3413.

[11] A.E. Kaifer, Functionalised self-assembled monolayers containing preformed binding sites, Israel Journal of Chemistry, 1996, 36, 3899-397.

[12] B. Piro & al., A polyamide film for dopamine entrapment and delivery, Journal of Electroanalitical Chemistry, 2001, 499, 103-111.

[13] C.J. Stanley (Affymetric), Electrochemical denaturation of double stranded nucleic acid, patent number US 6 395 489 B1, **May 28, 2002.**

[14] J. Wang & al., On-demand electrochemical release of DNA from gold surfaces, Bioelectrochemistry, 2000, 52, 111-114.

[15] FR-A-2 818 287.

[16] US 5,919,523.

[17] WO-A-02/051856.

[18] FR-A-2 103 359.

[19] J. Voldmann, et al, Microfabrication in biology and medecine ; Annu. Rev. Biomed Eng., 1999, 01: 401-425.

[20] Hofmann F., et al, (Infineon Technologies); Fully electronic DNA détection on a CMOS chip: device and process issues ; Electron Devices Meeting ; 2002. IEDM '02. Digest. International ; 2002 Page (s): 488 -491.

[21] Godillot P. & al, Synthetic Materials, 1996, 83, pp117-123.

[22] Baueler P et al, Adv. Mat., 1996, 8, 3, pp214.

**Revendications**

1. Dispositif comprenant :

   - un support ayant une surface comportant une zone d'accrochage (Z) susceptible d'être fonctionnalisée par une sonde (A) capable de se lier à une cible (B) pour l'accrocher ;
   - une électrode de travail (WE) et une contre électrode (CE) de cette électrode de travail disposées sur le support au voisinage de la zone d'accrochage,

   dans lequel l'électrode de travail borde ou entoure la zone d'accrochage ;

   - des moyens permettant d'appliquer un courant électrique ou un potentiel déterminé à ladite électrode de travail de manière à provoquer, lorsque ladite zone d'accrochage et lesdites électrodes sont immergées dans une solution aqueuse, une variation locale de pH au niveau de ladite zone d'accrochage ;

   ladite zone d'accrochage (Z) et ladite électrode de travail (WE) étant séparées l'une de l'autre par un espace vide.

2. Dispositif selon la revendications 1, dans lequel ledit espace entre ladite zone d'accrochage (Z) et ladite électrode de travail (WE) est de 10 à 200 $\mu$m.

3. Dispositif selon la revendication 1, comprenant en outre une électrode de référence placée de manière à pouvoir mesurer le potentiel appliqué à l'électrode de travail.

4. Dispositif selon la revendication 3, dans lequel

   - ladite zone d'accrochage (Z) présente un diamètre de 1 à 1000 $\mu$m ;
   - l'espace entre ladite zone d'accrochage (Z) et ladite électrode de travail (WE) et l'espace entre l'électrode de travail (WE) et la contre électrode (CE) est un espace de 10 à 200 $\mu$m ;
   - la largeur de l'électrode de travail (WE) et de la contre électrode (CE) est comprise entre 10 et 50 $\mu$m ; et
   - l'électrode de référence (RE) est un parallélépipède de 10 X 500 $\mu$m.

5. Dispositif selon l'une quelconque des revendications 1 à 4, **caractérisé en ce que** ledit dispositif comprend

   - un support ayant une surface comportant une zone d'accrochage (Z) se présentant sous forme d'un disque compris dans un plan de référence, susceptible d'être fonctionnalisée par une sonde (A) capable de se lier à une cible (B) pour l'accrocher ;
   - une électrode de travail (WE) présentant, dans ledit plan de référence, une partie annulaire fermée qui entoure la zone d'accrochage (Z) et qui se prolonge par une partie rectangulaire, ladite électrode de travail (WE) et ladite zone d'accrochage (Z) étant séparées l'une de l'autre par un espace ;
   - une contre électrode (CE) présentant, dans ledit plan de référence, une partie annulaire ouverte qui entoure la partie annulaire de l'électrode de travail (WE) et qui se prolonge par une partie rectangulaire qui borde un des côtés de la partie rectangulaire de ladite électrode de travail (WE), ladite électrode de travail (WE) et ladite contre électrode (CE) étant séparées l'une de l'autre par un espace ;

- une électrode de référence (RE) de forme rectangulaire dans ledit plan de référence qui borde l'autre côté de la partie rectangulaire de ladite électrode de travail (WE), ladite électrode de travail (WE) et ladite électrode de référence (RE) étant séparées l'une de l'autre par un espace.

6. Dispositif selon l'une quelconque des revendications 1 à 4, **caractérisé en ce que** ledit dispositif comprend

- un support ayant une surface comportant une zone d'accrochage (Z) se présentant sous forme d'un disque compris dans un plan de référence prolongé par une partie rectangulaire, susceptible d'être fonctionnalisée par une sonde (A) capable de se lier à une cible (B) pour l'accrocher ;

- une électrode de travail (WE) présentant, dans ledit plan de référence, une partie annulaire ouverte qui entoure la partie sous forme d'un disque de la zone d'accrochage (Z) et qui se prolonge par une partie rectangulaire qui borde la partie rectangulaire de la zone d'accrochage (Z), ladite électrode de travail (WE) et ladite zone d'accrochage (Z) étant séparées l'une de l'autre par un espace ;

- une contre électrode (CE) présentant, dans ledit plan de référence, une partie annulaire ouverte qui entoure la partie annulaire de l'électrode de travail (WE) et qui se prolonge par une partie rectangulaire qui borde l'autre côté de la partie rectangulaire de ladite zone d'accrochage (Z), ladite électrode de travail (WE) et ladite contre électrode (CE) étant séparées l'une de l'autre par un espace ;

- une électrode de référence (RE) de forme rectangulaire dans ledit plan de référence qui borde l'autre côté de la partie rectangulaire de ladite électrode de travail (WE), ladite électrode de travail (WE) et ladite électrode de référence (RE) étant séparées l'une de l'autre par un espace.

7. Dispositif selon la revendication 1, dans lequel l'électrode de travail borde ou entoure la zone d'accrochage, et la contre électrode borde ou entoure ladite électrode de travail.

8. Dispositif selon la revendication 1, dans lequel l'électrode de travail, la contre électrode et la zone d'accrochage sont dans une configuration choisie dans le groupe constitué d'une configuration de peigne inter-digité, d'une configuration spirale et d'une configuration concentrique.

9. Dispositif selon la revendication 1, dans lequel les moyens permettant d'appliquer un courant électrique ou un potentiel déterminé à ladite électrode de travail sont des moyens permettant d'appliquer une (ou des) impulsion(s) de courant(s) ou de potentiel(s) de durée, de valeur et de forme spécifiques et indépendante(s) les unes des autres.

10. Dispositif selon la revendication 1, dans lequel la zone d'accrochage est sous la forme d'une électrode.

11. Dispositif selon la revendication 1, dans lequel la zone d'accrochage est fonctionnalisée par une sonde (A) capable de se lier, en fonction du pH, à la cible (B) pour l'accrocher.

12. Dispositif selon la revendication 11, dans lequel la sonde est telle qu'elle est capable de se lier à la cible pour l'accrocher au moyen d'un groupement électrophile ou nucléophile.

13. Dispositif selon la revendication 11, dans lequel la sonde est telle qu'elle est capable de se lier à la cible pour l'accrocher au moyen d'un groupement électrophile choisi dans le groupe constitué des fonctions aldéhyde, halogénure, thiocyanate, isocyanate, ester activé, carbamate, et époxyde.

14. Dispositif selon la revendication 11, dans lequel la sonde est telle qu'elle est capable de se lier à la cible pour l'accrocher au moyen d'un groupement nucléophile choisi dans le groupe constitué des fonctions amine, alcoolate, phénol, phénate, oxyamine et hydrazine.

15. Dispositif selon la revendication 11, dans lequel la sonde est choisie de manière à ce qu'elle puisse former, dans la solution de travail, avec la molécule cible pour l'accrocher, une liaison choisie dans le groupe constitué d'une liaison hydrogène, peptidique, amide, sulfonamide, ester d'acide carboxylique, ester d'acide sulfonique ou silanoate substitué.

16. Dispositif selon la revendication 11, dans lequel la zone d'accrochage est fonctionnalisée par une sonde choisie dans le groupe constitué d'un oligonucléotide, une protéine, un enzyme, un substrat d'enzyme, un récepteur hormonal, une hormone, un anticorps, un antigène, une cellule eucaryote ou procaryote ou des fragments de telles cellules, une algue ou un champignon microscopique.

17. Microsystème électrochimique **caractérisé en ce qu'**il comprend un ou plusieurs dispositif(s) selon l'une quelconque des revendications 1 à 16.

**18.** Utilisation d'un dispositif selon l'une quelconque des revendications 1 à 16 dans un procédé destiné à purifier, à concentrer, à cribler ou à détecter une cible ou un objet.

**19.** Procédé d'accrochage d'une cible (B) présente dans un échantillon aqueux à une sonde (A), ledit procédé comprenant les étapes suivantes :

a) mise en contact de l'échantillon aqueux avec la zone d'accrochage d'un dispositif selon la revendication 1 fonctionnalisée par la sonde (A) capable de se lier, en fonction du pH, à la cible (B) pour l'accrocher ;
b) application d'un courant électrique ou d'un potentiel à l'électrode de travail dudit dispositif de façon à modifier localement, au niveau de ladite zone d'accrochage, le pH de l'échantillon aqueux pour que la sonde reconnaisse et se lie spécifiquement à la cible pour l'accrocher.

**20.** Procédé d'accrochage et de décrochage d'une cible (B) présente dans un échantillon aqueux sur une sonde (A), ledit procédé comprenant les étapes suivantes :

a') mise en contact de l'échantillon aqueux comprenant la cible (B) avec la zone d'accrochage d'un dispositif selon la revendication 1 fonctionnalisée par la sonde (A) pour que la cible (B) s'accroche à ladite sonde ;
b') application d'un courant électrique ou d'un potentiel à l'électrode de travail dudit dispositif de façon à modifier localement, au niveau de ladite zone d'accrochage, le pH de la solution de travail pour que la cible (B) se décroche de la sonde (A).

**21.** Procédé selon la revendication 20, dans lequel l'accrochage de la cible par la sonde dans l'étape a') est réalisé par application d'un courant électrique ou d'un potentiel à l'électrode de travail dudit dispositif de façon à modifier localement, au niveau de ladite zone d'accrochage, le pH de la solution de travail pour que la cible (B) s'accroche à ladite sonde (A).

**22.** Procédé selon la revendication 19 ou 20, comprenant en outre l'étape suivante, avant ou après l'accrochage de la cible par la sonde :

(x) fixation d'un objet sur la cible.

**23.** Procédé selon la revendication 22, dans lequel l'objet est choisi dans le groupe constitué d'une molécule, d'une cellule; d'une bactérie ; de billes fonctionnalisées ; d'une protéine ; un oligonucléotide ; d'une enzyme ; d'un anticorps ; d'un fragment biologique ; de molécules à transfecter; de molécules d'intérêt biologique ; de principes actifs ; de molécules d'intérêt-pharmacologique.

**24.** Procédé selon la revendication 22, dans lequel l'objet est un marqueur permettant de détecter la cible, ledit procédé comprenant en outre un étape de détection de la cible marquée.

**25.** Procédé selon la revendication 19 ou 20, dans lequel l'échantillon comprenant la cible est sous forme d'une solution aqueuse tamponnée.

**26.** Procédé selon la revendication 19 ou 20, dans lequel la cible et la sonde sont des oligonucléotide complémentaires entre eux.

**27.** Procédé selon la revendication 24, dans lequel la sonde porte une biotine, et la cible est marquée par de la streptavidine-phycoérythrine.

**28.** Procédé selon la revendication 19 ou 20, dans lequel ledit dispositif est un dispositif selon l'une quelconque des revendications 11 à 17.

**29.** Utilisation d'un procédé selon l'une quelconque des revendications 19 à 27 dans un procédé destiné à extraire, à concentrer, à cribler ou à détecter une cible ou un objet.

**30.** Utilisation selon la revendication 29, dans laquelle la cible ou l'objet est choisi dans le groupe constitué d'un oligonucléotide, une protéine, un enzyme, un substrat d'enzyme, un récepteur hormonal, une hormone, un anticorps, un antigène, une cellule eucaryote ou procaryote ou des fragments de telles cellules, une algue ou un champignon microscopique.

**Claims**

**1.** Device comprising:

- a support having a surface comprising an attachment zone (Z) able to be functionalised by a sensor (A) capable of binding to a target (B) in order to attach it;
- a working electrode (WE) and a counter-electrode (CE) for this working electrode both disposed on the support in the vicinity of the attachment zone, in which the working electrode borders or surrounds the attachment zone;
- means for applying an electric current or a given potential to the said working electrodes so as to cause, when the said attachment zone and the said electrodes are immersed in an aqueous solution, a local variation in pH at said attachment zone;

said attachment zone (Z) and said working electrode (WE) being separated from each other by an empty space.

2. Device according to claim 1, in which said space between said attachment zone (Z) and said working electrode (WE) is 10 to 200 μm.

3. Device according to claim 1, also comprising a reference electrode placed so as to be able to measure the potential applied to the working electrode.

4. Device according to claim 3, in which

    - said attachment zone (Z) has a diameter of 1 to 1000 μm;
    - the space between said attachment zone (Z) and said working electrode (WE) and the space between the working electrode (WE) and the counter-electrode (CE) is a space of the 10 to 200 μm;
    - the width of the working electrode (WE) and of the counter-electrode (CE) is between 10 and 50 μm; and
    - the reference electrode (RE) is a parallelepiped of 10 x 500 μm.

5. Device according to any one of claims 1 to 4, **characterised in that** said device comprises

    - a support having a surface comprising an attachment zone (Z) in the form of a disc included in a reference plane, able to be functionalised by a sensor (A) capable of binding to a target (B) in order to attach it;
    - a working electrode (WE) having, in said reference plane, a closed annular part that surrounds the attachment zone (Z) and is extended by a rectangular part, said working electrode (WE) and said attachment zone (Z) being separated from each other by a space;
    - a counter-electrode (CE) having, in said reference plane, and open annular part that surrounds the annular part of the working electrode (WE) and is extended by a rectangular part that borders one of the sides of the rectangular part of said working electrode (WE), said working electrode (WE) and said counter-electrode (CE) being separated from each other by a space;
    - a rectangular-shaped reference electrode (RE) in said reference plane that borders the other side of the rectangular part of said working electrode (WE), said working electrode (WE) and said reference electrode (RE) being separated from each other by a space.

6. Device according to any one of claims 1 to 4, **characterised in that** said device comprises

    - a support having a surface comprising an attachment zone (Z) in the form of a disc included in a reference plane extended by a rectangular part, able to be functionalised by a sensor (A) capable of binding to a target (B) in order to attach it;
    - a working electrode (WE) having, in said reference plane, an open annular part that surrounds the disc-shaped part of the attachment zone (Z) and is extended by a rectangular part that borders the rectangular part of the attachment zone (Z), said working electrode (WE) and said attachment zone (Z) being separated from each other by a space;
    - a counter-electrode (CE) having, in said reference plane, an open annular part that surrounds the annular part of the working electrode (WE) and is extended by a rectangular part that borders the other side of the rectangular part of said attachment zone (Z), said working electrode (WE) and said counter electrode (CE) being separated from each other by a space;
    - a rectangular-shaped reference electrode (RE) in said reference plane that borders the other side of the rectangular part of said working electrode (WE), said working electrode (WE) and said reference electrode (RE) being separated from each other by a space.

7. Device according to Claim 1, in which the working electrode borders or surrounds the attachment zone, and the counter-electrode borders or surrounds said working electrode.

8. Device according to Claim 1, in which the working electrode, the counter-electrode and the attachment zone are in a design chosen from the group consisting of an interdigitated comb design, a spiral design and a concentric design.

9. Device according to Claim 1, in which the means for applying a given electric current or a given potential to said working electrode are means for applying one (or more) current or potential pulse(s) with a time period, a value and a form that are specific to it(them) and that is(are) independent from each other.

10. Device according to Claim 1, in which the attachment zone is in the form of an electrode.

11. Device according to Claim 1, in which the attachment zone is functionalized with the probe (A) capable of binding, according to the pH, to the target (B) so as to attach it.

12. Device according to Claim 11, in which the probe is such that it is capable of binding to the target so as to attach it by means of an electrophilic or nucleophilic group.

13. Device according to Claim 11, in which the probe is such that it is capable of binding to the target so as to attach it by means of an electrophilic group chosen from the group consisting of aldehyde, halide, thiocyanate, isocyanate, activated ester, carbamate and epoxide functions.

14. Device according to Claim 11, in which the probe is such that it is capable of binding to the target so as to attach it by means of a nucleophilic group chosen from the group consisting of amine, alkoxide, phenol, phenate, oxyamine and hydrazine functions.

15. Device according to Claim 11, in which the probe is chosen such that it can form, in the working solution, with the target molecule so as to attach it, a bond chosen from the group consisting of a hydrogen, peptide, amide, sulphonamide, carboxylic acid ester, sulphonic acid ester or substituted silanoate bond.

16. Device according to Claim 11, in which the attachment zone is functionalized with a probe chosen from the group consisting of an oligonucleotide, a protein, an enzyme, an enzyme substrate, a hormone receptor, a hormone, an antibody, an antigen, a eukaryotic or prokaryotic cell or fragments of such cells, an alga or a microscopic fungus.

17. Electrochemical microsystem, **characterized in that** it comprises one or more device(s) according to any one of Claims 1 to 16.

18. Use of a device according to any one of Claims 1 to 16, in a method intended to purify, concentrate, screen or detect a target or an object.

19. Method for attaching a target (B) present in an aqueous sample to a probe (A), said method comprising the following steps:

    a) bringing the aqueous sample into contact with the attachment zone of a device according to Claim 1, functionalized with the probe (A) capable of binding, according to the pH, to the target (B) so as to attach it;
    b) applying an electric current or a potential to the working electrode of said device so as to locally modify, in the region of said attachment zone, the pH of the aqueous sample such that the probe recognizes and binds specifically to the target so as to attach it.

20. Method for attaching and detaching a target (B) present in an aqueous sample to and from a probe (A), said method comprising the following steps:

a') bringing the aqueous sample comprising the target (B) into contact with the attachment zone of a device according to Claim 1, functionalized with the probe (A), such that the target (B) attaches to said probe;
b') applying an electric current or potential to the working electrode of said device so as to locally modify, in the region of said attachment zone, the pH of the working solution such that the target (B) detaches from the probe (A).

21. Method according to Claim 20, in which the attachment of the target by the probe in step a') is carried out by applying an electric current or a potential to the working electrode of said device so as to locally modify, in the region of said attachment zone, the pH of the working solution such that the target (B) attaches to said probe (A).

22. Method according to Claim 19 or 20, also comprising the following step, before or after the attachment of the target by the probe:

    (x) attachment of an object to the target.

23. Method according to Claim 22, in which the object is chosen from the group consisting of a molecule, a cell; a bacterium; functionalized beads; a protein; an oligonucleotide; an enzyme; an antibody; a biological fragment; molecules to be transfected; molecules of biological interest; active principles; molecules of pharmacological interest.

24. Method according to Claim 22, in which the object is a label for detecting the target, said method also comprising a step consisting in detecting the labelled target.

25. Method according to Claim 19 or 20, in which the sample comprising the target is in the form of a buffered aqueous solution.

26. Method according to Claim 19 or 20, in which the target and the probe are oligonucleotides complementary to one another.

27. Method according to Claim 24, in which the probe carries a biotin, and the target is labelled with streptavidin-phycoerythrin.

28. Method according to Claim 19 or 20, in which said device is a device according to any one of Claims 11 to 17.

29. Use of a method according to any one of Claims 19 to 27, in a method intended for extracting, concentrating, screening or detecting a target or an object.

**30.** Use according to Claim 29, in which the target or the object is chosen from the group consisting of an oligonucleotide, a protein, an enzyme, an enzyme substrate, a hormone receptor, a hormone, an antibody, an antigen, a eukaryotic or prokaryotic cell or fragments of such cells, an alga or a microscopic fungus.

**Patentansprüche**

**1.** Vorrichtung umfassend:

- einen Träger mit einer Oberfläche, die eine Haftzone (Z) umfasst, die durch eine Sonde (A) funktionalisiert werden kann, die zum Binden an ein Ziel (B) unter seinem Anhaften befähigt ist,
- eine Arbeitselektrode (WE) und eine Gegenelektrode (CE) zu dieser Arbeitselektrode, die auf dem Träger in der Nähe der Haftzone angeordnet sind, wobei die Arbeitselektrode die Haftzone einfasst oder umgibt,
- Mittel, die das Anlegen eines vorgegebenen elektrischen Stroms oder eines Potentials an die Arbeitselektrode ermöglichen, um eine örtliche Änderung des pH im Bereich der Haftzone zu bewirken, wenn die Haftzone und die Elektroden in eine wässrige Lösung eingetaucht werden,

wobei die Haftzone (Z) und die Arbeitselektrode (WE) durch einen Leerraum voneinander getrennt sind.

**2.** Vorrichtung gemäß Anspruch 1, wobei der Zwischenraum zwischen der Haftzone (Z) und der Arbeitselektrode (WE) 10 bis 200 $\mu$m beträgt.

**3.** Vorrichtung gemäß Anspruch 1, die außerdem eine Bezugselektrode umfasst, die so angebracht ist, dass sie das an die Arbeitselektrode angelegte Potential messen kann.

**4.** Vorrichtung gemäß Anspruch 3, wobei

- die Haftzone (Z) einen Durchmesser von 1 bis 1000 $\mu$m aufweist,
- der Raum zwischen der Haftzone (Z) und der Arbeitselektrode (WE) und der Raum zwischen der Arbeitselektrode (WE) und der Gegenelektrode (CE) ein Zwischenraum von 10 bis 200 $\mu$m ist,
- die Breite der Arbeitselektrode (WE) und der Gegenelektrode (CE) zwischen 10 und 50 $\mu$m beträgt und
- die Bezugselektrode (RE) ein Parallelepiped von 10 x 500 $\mu$m ist.

**5.** Vorrichtung gemäß einem der Ansprüche 1 bis 4, **dadurch gekennzeichnet, dass** die Vorrichtung Folgendes umfasst:

- einen Träger mit einer Oberfläche, die eine Haftzone (Z) umfasst, die in Form einer von einer Bezugsebene umfassten Scheibe vorliegt und durch eine Sonde (A) funktionalisiert werden kann, die zum Binden an ein Ziel (B) unter seinem Anhaften befähigt ist,
- eine Arbeitselektrode (WE), die in dieser Bezugsebene einen geschlossenen, ringförmigen Teil aufweist, der die Haftzone (Z) umgibt und der sich durch einen rechteckigen Teil verlängert, wobei die Arbeitselektrode (WE) und die Haftzone (Z) durch einen Zwischenraum voneinander getrennt sind,
- eine Gegenelektrode (CE), die in dieser Bezugsebene einen offenen, ringförmigen Teil aufweist, der den ringförmigen Teil der Arbeitselektrode (WE) umgibt und der sich durch einen rechteckigen Teil verlängert, der eine der Seiten des rechteckigen Teils der Arbeitselektrode (WE) einfasst, wobei die Arbeitselektrode (WE) und die Gegenelektrode (CE) durch einen Zwischenraum voneinander getrennt sind,
- eine Bezugselektrode (RE) mit rechteckiger Form in der Bezugsebene, die die andere Seite des rechteckigen Teils der Arbeitselektrode (WE) einfasst, wobei die Arbeitselektrode (WE) und die Bezugselektrode (RE) durch einen Zwischenraum voneinander getrennt sind.

**6.** Vorrichtung gemäß einem der Ansprüche 1 bis 4, **dadurch gekennzeichnet, dass** die Vorrichtung Folgendes umfasst:

- einen Träger mit einer Oberfläche, die eine Haftzone (Z) umfasst, die in Form einer von einer Bezugsebene umfassten Scheibe vorliegt, die durch einen rechteckigen Teil verlängert wird und durch eine Sonde (A) funktionalisiert werden kann, die zum Binden an ein Ziel (B) unter seinem Anhaften befähigt ist,
- eine Arbeitselektrode (WE), die in dieser Bezugsebene einen offenen, ringförmigen Teil aufweist, der den Teil in Form einer Scheibe der Haftzone (Z) umgibt und der sich durch einen rechteckigen Teil verlängert, der den rechteckigen Teil der Haftzone (Z) einfasst, wobei die Arbeitselektrode (WE) und die Haftzone (Z) durch einen Zwischenraum voneinander getrennt sind,
- eine Gegenelektrode (CE), die in dieser Bezugsebene einen offenen, ringförmigen Teil aufweist, der den ringförmigen Teil der Arbeitselektrode (WE) umgibt und der sich durch einen rechteckigen Teil verlängert, der die andere Seite des rechteckigen Teils der Haftzone (Z) einfasst, wobei die Arbeitselektrode (WE) und die Gegenelektrode (CE) durch einen Zwischenraum voneinander getrennt sind,
- eine Bezugselektrode (RE) mit rechteckiger Form in der Bezugsebene, die die andere Seite des rechteckigen Teils der Arbeitselektrode (WE) einfasst, wobei die Arbeitselektrode (WE) und die Bezugselektrode (RE) durch einen Zwischenraum voneinander getrennt sind.

7. Vorrichtung gemäß Anspruch 1, bei der die Arbeitselektrode die Haftzone einfasst oder umgibt und die Gegenelektrode die Arbeitselektrode einfasst oder umgibt.

8. Vorrichtung gemäß Anspruch 1, bei der sich die Arbeitselektrode, die Gegenelektrode und die Haftzone in einer Anordnung befinden, die aus der aus einer Anordnung eines verzahnten Kamms, einer spiralförmigen Anordnung und einer konzentrischen Anordnung bestehenden Gruppe ausgewählt ist.

9. Vorrichtung gemäß Anspruch 1, bei der die das Anlegen eines vorgegebenen elektrischen Stroms oder eines Potentials an die Arbeitselektrode ermöglichenden Mittel Mittel sind, die das Anlegen eines oder mehrerer, voneinander unabhängiger Strom- oder Potentialpulse von spezieller Dauer, Größe und Form ermöglichen.

10. Vorrichtung gemäß Anspruch 1, bei der die Haftzone in Form einer Elektrode vorliegt.

11. Vorrichtung gemäß Anspruch 1, bei der die Haftzone durch eine Sonde (A) funktionalisiert ist, die als Funktion des pH zum Binden an das Ziel (B) unter seinem Anhaften befähigt ist.

12. Vorrichtung gemäß Anspruch 11, bei der die Sonde solcherart ist, dass sie zum Binden an das Ziel unter seinem Anhaften mittels einer elektrophilen oder nukleophilen Gruppe befähigt ist.

13. Vorrichtung gemäß Anspruch 11, bei der die Sonde solcherart ist, dass sie zum Binden an das Ziel unter seinem Anhaften mittels einer elektrophilen Gruppe befähigt ist, die aus der aus Aldehyd-, Halogen-, Thiocyanat-, Isocyanat-, aktivierten Ester-, Carbamat- und Epoxidfunktionen bestehenden Gruppe ausgewählt ist.

14. Vorrichtung gemäß Anspruch 11, bei der die Sonde solcherart ist, dass sie zum Binden an das Ziel unter seinem Anhaften mittels einer nukleophilen Gruppe befähigt ist, die aus der aus Amin-, Alkoholat-, Phenol-, Phenat-, Oxyamin- und Hydrazinfunktionen bestehenden Gruppe ausgewählt ist.

15. Vorrichtung gemäß Anspruch 11, bei der die Sonde so ausgewählt ist, dass sie in der Arbeitslösung mit dem Zielmolekül unter seinem Anhaften eine Bindung ausbilden kann, die aus der aus einer Wasserstoff-, Peptid-, Amid-, Sulfonamid-, Carbonsäureester-, Sulfonsäureester- und substituierten Silanoatbindung bestehenden Gruppe ausgewählt ist.

16. Vorrichtung gemäß Anspruch 11, bei der die Haftzone durch eine Sonde funktionalisiert ist, die aus der aus einem Oligonukleotid, einem Protein, einem Enzym, einem Enzymsubstrat, einem hormonellen Rezeptor, einem Hormon, einem Antikörper, einem Antigen, einer Eukaryonten- oder Prokaryontenzelle oder Fragmenten derartiger Zellen, einer Alge oder einem mikroskopischen Pilz bestehenden Gruppe ausgewählt ist.

17. Elektrochemisches Mikrosystem, **dadurch gekennzeichnet, dass** es eine oder mehrere Vorrichtungen gemäß einem der Ansprüche 1 bis 16 umfasst.

18. Verwendung einer Vorrichtung gemäß einem der Ansprüche 1 bis 16 in einem zum Reinigen, Konzentrieren, Durchmustern oder Nachweisen eines Ziels oder eines Objekts bestimmten Verfahren.

19. Verfahren zum Anhaften eines in einer wässrigen Probe vorliegenden Ziels (B) an eine Sonde (A), wobei das Verfahren die folgenden Schritte umfasst:

a) In-Kontakt-Bringen der wässrigen Probe mit der Haftzone einer Vorrichtung gemäß Anspruch 1, die durch die Sonde (A) funktionalisiert ist, die als Funktion des pH zum Binden an das Ziel (B) unter seinem Anhaften befähigt ist,
b) Anlegen eines elektrischen Stroms oder eines Potentials an die Arbeitselektrode dieser Vorrichtung, so dass der pH der wässrigen Probe im Bereich der Haftzone örtlich verändert wird, damit die Sonde das Ziel erkennt und unter seinem Anhaften spezifisch bindet.

20. Verfahren zum Anhaften und Entfernen eines in einer wässrigen Probe vorliegenden Ziels (B) auf einer Sonde (A), wobei das Verfahren die folgenden Schritte umfasst:

a') In-Kontakt-Bringen der das Ziel (B) umfassenden wässrigen Probe mit der Haftzone einer Vorrichtung gemäß Anspruch 1, die durch die Sonde (A) funktionalisiert ist, damit das Ziel (B) an der Sonde haftet,
b') Anlegen eines elektrischen Stroms oder eines Potentials an die Arbeitselektrode der Vorrichtung, so dass der pH der Arbeitslösung im Bereich dieser Zone örtlich verändert wird, damit das Ziel (B) von der Sonde (A) entfernt wird.

**21.** Verfahren gemäß Anspruch 20, wobei das Anhaften des Ziels durch die Sonde in Schritt a') durch Anlegen eines elektrischen Stroms oder eines Potentials an die Arbeitselektrode der Vorrichtung bewerkstelligt wird, so dass der pH der Arbeitslösung im Bereich der Haftzone örtlich verändert wird, damit das Ziel (B) an der Sonde (A) haftet.

**22.** Verfahren gemäß Anspruch 19 oder 20, das außerdem vor oder nach dem Anhaften des Ziels durch die Sonde den folgenden Schritt umfasst:

   (x) Fixieren eines Objekts auf dem Ziel.

**23.** Verfahren gemäß Anspruch 22, wobei das Objekt aus der aus einem Molekül, einer Zelle, einem Bakterium, funktionalisierten Kugeln, einem Protein, einem Oligonukleotid, einem Enzym, einem Antikörper, einem biologischen Fragment, zu transfizierenden Molekülen, Molekülen von biologischem Interesse, aktiven Prinzipien und Molekülen von pharmakologischem Interesse bestehenden Gruppe ausgewählt ist.

**24.** Verfahren gemäß Anspruch 22, wobei das Objekt ein Marker ist, der den Nachweis des Ziels ermöglicht, und das Verfahren außerdem einen Schritt des Nachweises des markierten Ziels umfasst.

**25.** Verfahren gemäß Anspruch 19 oder 20, wobei die das Ziel umfassende Probe in Form einer gepufferten, wässrigen Lösung vorliegt.

**26.** Verfahren gemäß Anspruch 19 oder 20, wobei das Ziel und die Sonde miteinander komplementäre Oligonukleotide sind.

**27.** Verfahren gemäß Anspruch 24, wobei die Sonde Biotin trägt und das Ziel durch Streptavidin-Phycoerythrin markiert ist.

**28.** Verfahren gemäß Anspruch 19 oder 20, wobei die Vorrichtung eine Vorrichtung gemäß einem der Ansprüche 11 bis 17 ist.

**29.** Anwendung eines Verfahrens gemäß einem der Ansprüche 19 bis 27 bei einem zum Extrahieren, Konzentrieren, Durchmustern oder Nachweisen eines Ziels oder eines Objekts bestimmten Verfahren.

**30.** Anwendung gemäß Anspruch 29, wobei das Ziel oder das Objekt aus der aus einem Oligonukleotid, einem Protein, einem Enzym, einem Enzymsubstrat, einem hormonellen Rezeptor, einem Hormon, einem Antikörper, einem Antigen, einer Eukaryonten- oder Prokaryontenzelle oder Fragmenten derartiger Zellen, einer Alge oder einem mikroskopischen Pilz bestehenden Gruppe ausgewählt ist.

FIG. 1

FIG. 2

FIG. 3

FIG. 4

FIG. 5

FIG. 6

FIG. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 02051856 A
- WO 02090963 A1
- EP 1072617 A1
- US 6395489 B1
- FR 2818287 A
- US 5919523 A
- FR 2103359 A

**Littérature non-brevet citée dans la description**

- **E. KATZ.** pH switched electrochemistry of pyrroloquinoline quinone at Au electrodes modified by functionalized monolayers. *Journal of Electroanalytical Chemistry,* 1996, vol. 408, 107-112
- Dynamic substrates : modulating the behaviours of attached cells. **M.N. YOUSAF ; M. MRKSICH.** New Technologies for life sciences : A trends guide. Elsevier, Décembre 2000, 28-35
- **V. CHECHIK.** Reactions and reactivity in SAMs. *Advanced Materials,* 2000, vol. 12 (16), 1161
- **E. KAGANER.** Surface Plasmon Resonance Characterization of Photoswitchable Antigen-Antibody Interactions. *Langmuir,* 1999, vol. 15 (11), 3920-3923
- **M. YAMATO et al.** Novel patterned cell coculture utilizing thermally responsive grafted polymer surfaces. *Journal of Biomedical Materials Research,* 2001, vol. 55 (1), 137-140
- **D.J. REVELL.** SA carbohydrate Ms : formation and surface selective molecular recognition. *Langmuir,* 1998, vol. 14, 4517-4524
- **J. SPINKE.** Molecular recognition at SAM : optimisation of surface fictionalisation. *Journal of Chemical Physics,* 1993, vol. 99 (9), 7012-019
- **C.D. TIDWELL.** Endothelial cell growth and protein adsorption on terminally functionalized, SAMs of alkanethiolates on gold. *Langmuir,* 1997, vol. 13, 3404-3413
- **A.E. KAIFER.** Functionalised self-assembled monolayers containing preformed binding sites. *Israel Journal of Chemistry,* 1996, vol. 36, 3899-397
- **B. PIRO.** A polyamide film for dopamine entrapment and delivery. *Journal of Electroanalitical Chemistry,* 2001, vol. 499, 103-111
- **J. WANG.** On-demand electrochemical release of DNA from gold surfaces. *Bioelectrochemistry,* 2000, vol. 52, 111-114
- **J. VOLDMANN et al.** Microfabrication in biology and medecine. *Annu. Rev. Biomed Eng.,* 1999, vol. 01, 401-425
- **HOFMANN F. et al.** Infineon Technologies); Fully electronic DNA détection on a CMOS chip: device and process issues ; Electron Devices Meeting. *2002. IEDM '02. Digest. International,* 2002, 488-491
- **GODILLOT P.** *Synthetic Materials,* 1996, vol. 83, 117-123
- **BAUELER P et al.** *Adv. Mat.,* 1996, vol. 8 (3), 214